(19) Europäisches Patentamt
European Patent Office
Office européen des brevets

(11) EP 4 249 578 A1

(12) EUROPEAN PATENT APPLICATION

(43) Date of publication:
**27.09.2023 Bulletin 2023/39**

(21) Application number: **22202167.7**

(22) Date of filing: **18.10.2022**

(51) International Patent Classification (IPC):
***C11D 1/75** (2006.01)* ***C11D 1/94** (2006.01)*
***C11D 11/04** (2006.01)* ***C07C 303/24** (2006.01)*
***C07C 305/06** (2006.01)*

(52) Cooperative Patent Classification (CPC):
(C-Sets available)
**C11D 1/94; C07C 303/24; C11D 11/04;** C11D 1/75

(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.03.2022 EP 22160488**

(71) Applicant: **The Procter & Gamble Company
Cincinnati, OH 45202 (US)**

(72) Inventors:
- **BRAECKMAN, Karl Ghislain
1853 Strombeek-Bever (BE)**
- **JONES, Christopher Stephen
1853 Strombeek-Bever (BE)**
- **KEULEERS, Robby Renilde Francois
1853 Strombeek-Bever (BE)**
- **STEPHANS, Scott Edward
Cincinnati, 45202 (US)**
- **TARCHINI, Rocco
1853 Strombeek-Bever (BE)**
- **TARIQ, Saima
1853 Strombeek-Bever (BE)**
- **VANHOUTTE, Diederik Emiel Omer
1853 Strombeek-Bever (BE)**

(74) Representative: **P&G Patent Belgium UK
N.V. Procter & Gamble Services Company S.A.
Temselaan 100
1853 Strombeek-Bever (BE)**

# (54) PROCESSES FOR MAKING CONCENTRATED SURFACTANT BLENDS

(57) The need for an alkyl sulfated anionic surfactant containing concentrated surfactant blend which does not require a high pH in order to be hydrolytically stable, and as such, can be used to form detergent compositions which do not comprise high levels of salts or require the addition of high levels of organic solvents or structurants in order to be stable, and have the desired viscosity, dissolution and foaming profile, is met by a process whereby a buffering surfactant is added before or during the neutralisation step of the alkyl sulfuric acid stream to form the alkyl sulfated anionic surfactant.

EP 4 249 578 A1

(52) Cooperative Patent Classification (CPC): (Cont.)

C-Sets
**C07C 303/24, C07C 305/06**

## Description

FIELD OF THE INVENTION

The present invention relates to processes for making concentrated surfactant blends, in particular, concentrated surfactant blends for use in making liquid detergent compositions, especially liquid hand dishwashing cleaning compositions.

BACKGROUND OF THE INVENTION

Liquid hand dishwashing detergent compositions are typically high sudsing and provide long-lasting suds, in addition to good grease removal, in order to provide a high level of consumer satisfaction. Generally, such detergent compositions comprise alkyl sulfated anionic surfactants as they provide both high, long-lasting suds during use, and good grease removal. Alkyl sulfated anionic surfactants include non-alkoxylated alkyl sulfate surfactants and alkoxylated alkyl sulfate surfactants such as ethoxylated alkyl sulfate surfactants.

Liquid hand dishwashing detergent compositions typically include co-surfactants as part of the surfactant system to further boost the cleaning and sudsing performance. Such co-surfactants can include amphoteric surfactants such as amine oxide surfactants, zwitterionic surfactants such as betaine surfactants, and mixtures thereof.

In order to connote "richness" of the formula to the user, the liquid dishwashing detergent composition should also have a high viscosity.

Such alkyl sulfated anionic surfactants (including alkoxylated and non-alkoxylated alkyl sulfate surfactants) are made by processes involving a sulfation step, followed by a neutralisation step.

Various reagents have been used for the sulfation step, with sulfur trioxide (SO3) being particularly preferred, at least partially due to its low cost. Such processes are typically carried out using a film reactor, such as an annular falling film reactor, or a multi-tube film reactor, such as the "Ballestra" reactor. In such processes, the SO3 is typically first diluted with air. Such air/SO3 sulfation processes are direct processes in which SO3 gas is diluted with very dry air and reacted directly with the alkyl alcohol feedstock. The reaction of gaseous SO3 with the alkyl alcohol is rapid and stoichiometric. Such processes are complicated by the possibility of side reactions, However, with tight process control, very high purity alkyl sulfate surfactants can be achieved. However, the reaction typically falls short of completion. Hence, some acid residue remains which needs to be neutralised in order to achieve the desired pH of the blend. The resultant blend hence comprises neutralised salt and unreacted alkyl alcohol. Both lead to a reduction in phase stability, especially at low temperatures.

Alternatively, sulfamic acid can be used in the sulfation step in order to form the ammonium salt. Sulfamic acid is a mild and specific sulfating reagent suitable for making ammonium neutralized alcohol ethoxylates. An advantage of sulfamic acid is that it selectively sulfates alcohol groups and will not sulfonate aromatic rings. Therefore, it is particularly suited for sulfation of alkyl phenol ethoxylates, in order to prevent formation of mixed sulfate-sulfonate compounds. An advantage of using sulfamic acid as a reagent is that the neutralised form of the sulfate surfactant is produced. However, sulfamic acid is an expensive reagent. Moreover, when using sulfamic acid, one can only make the ammonium neutralized salt of the alkyl sulfate surfactant. This limits the application of the surfactants made using this sulfating reagent since the pH of compositions comprising such ammonium salts of alkyl sulfate surfactant has to be less than 8 in order to avoid an ammonia smell from the detergent composition. Alternatively, ion-exchange processes can be used to replace the ammonium ions with sodium or other ions. However, such ion exchange processes are expensive and typically not cost effective for applications such as household detergent compositions. Moreover, such processes result in low surfactant concentrations in the resultant blends of typically less than 30%, resulting in limiting their application to low active detergent compositions and higher transport costs for the blends.

Chlorosulfuric acid (ClSO3H) can also be used to produce alkyl sulfates and alkyl ether sulfates. While substantially cheaper than sulfamic acid, chlorosulfuric acid is still substantially more expensive than other reagents. In addition, additional equipment and complexity are added to the process by using such reagents, especially since as the reaction moves to completion, hydrochloric acid (HCl) is released. This acid must be scrubbed or otherwise recovered.

For most commercial processes used to make alkyl sulfate surfactants, such as the air/SO3 processes and processes using chlorosulfuric acid, the acid form of the alkyl sulfate surfactant is formed. However, such acid forms of the alkyl sulfate surfactant are not stable and have to be neutralised shortly after the sulfation step, since hydrolysis back to the alkyl alcohol and the starting acid is rapid at a pH of below 7. Sodium hydroxide is the most commonly neutralising agent used in the neutralising step. However, other neutralising agents such as potassium hydroxide, ammonia, mono-ethanolamine, di-ethanolamine, and tri-ethanolamine, amongst others, can be used. An excess of the neutralising agent is typically added during the neutralising step to reduce hydrolysis of the neutralized alkyl sulfuric salt back to the alkyl alcohol and sulfuric acid. However, some hydrolysis still occurs, leading to a drift downward in the pH since such prior art alkyl sulfate blends are only weakly buffered or even not buffered at all. The resultant increase in

sulfuric acid present auto-catalyses the hydrolysis reaction, leading to further and more rapid reduction in the pH as more of the acid is formed. As a result, alkyl sulfate blends which have been neutralised using sodium hydroxide or other alkali metal hydroxide are typically neutralised to a high pH of between 11.0 and 13.0.

Typically, alkyl sulfate containing surfactant blends are used to make detergent compositions having a pH of from 7.0 to 9.0. As such, the pH of the detergent composition has to be "trimmed" using an acid, such as citric acid or HCl. As a result, high levels of salts are typically present in the composition, which cause both viscosity and phase stability challenges, especially at low temperatures. Therefore, more organic solvent or structurants must be added in order to provide the desired low temperature stability and viscosity profile. Since high salt levels and high solvent levels can affect the solubility of other actives, this also makes formulation of the finished liquid detergent composition more complex. High salt levels also make the detergent composition harder to dissolve, leading to greater dissatisfaction from users

Prior to sulfation, the alkyl alcohol can be alkoxylated, especially ethoxylated. The resultant alkyl alkoxylated sulfate surfactants provide improved low temperature stability for the resultant liquid detergent composition, while also providing the desired level of grease cleaning and sudsing performance. When making alkoxylated, and especially ethoxylated alkyl sulfate surfactants, 1,4-dioxanes can be produced. Tight control of processing conditions and feedstock material compositions is typically needed, both during alkoxylation especially ethoxylation and sulfation steps, so that the amount of 1,4-dioxane by-product within alkoxylated especially ethoxylated alkyl sulfates can be minimised. Even if the 1,4-dioxane by-product level is kept to a minimum in the freshly formed surfactant blend, for many blends formed from prior art processes, the level of 1,4-dioxane by-product increases over time. The formation of 1,4-dioxanes has been found to be higher when concentrated alkyl ethoxylated sulfate blends are stored at higher pH.

As such, there remains a need for processes for forming alkyl sulfated anionic surfactant containing concentrated surfactant blends which do not require a high pH in order to be hydrolytically stable, and as such, can be used to form detergent compositions which do not comprise high levels of salts or require the addition of high levels of organic solvents or structurants in order to be stable and have the desired viscosity profile. A further need remains for a process for making concentrated surfactant blends which are more stable, especially at lower temperatures. In addition, there remains a need for a process for forming alkyl alkoxylated sulfate containing concentrated surfactant blends which comprise low levels of 1,4-dioxane by-product and in which the 1,4-dioxane by-product does not increase substantially over time.

US4477372A, US4476044A, and US4476045A relate to high active content surfactant wherein the anionic portion of the surfactant is neutralized with a secondary or tertiary amine containing at least three carbon atoms attached to the nitrogen atom of the amine, at least one alcoholic hydroxy group therein and such that the amine is alpha or beta substituted with respect to the nitrogen atom products. WO9418160A relates to a process for producing high active alkyl sulfate solutions comprising the steps of adding and mixing an alkyl sulfuric acid having a chain length of C12-C18, with an organic amine to produce a neutralized product having substantially no water. EP2964741A relates to a method of preparing a substantially anhydrous composition of alkyl (ethoxy) sulfate neutralized with an organic amine base, the said composition being suitable for use as a surfactant in an ecodose. WO201472840A relates to a process for preparing high-concentration, flowable aqueous fatty alkyl sulfate solution, said process comprises (i) ethoxylation of fatty alcohol with very low of about 0.3 to about 0.8 moles of ethylene oxide, and (ii) sulfating the ethoxylated fatty alcohol with specific reaction conditions, and (iii) neutralizing the sulfation product with an aqueous base, the obtained fatty alkyl sulfate solution contains at least 65% by weight of mixture of fatty alkyl sulfates and fatty alkyl ether sulfates in a weight ratio in the range from about 80:20 to about 50:50 wherein the average number of moles of ethylene oxide (EO) of the mixture is between 0.3 to 0.8; less than 3 ppm of dioxane; and water, and wherein the solution does not contain any antimicrobial or preservatives and is homogeneous, flowable and pumpable at 25°C. WO9738972A relates to sulfation methods for producing longer chain length alkyl sulfate and/or alkyl alkoxylated sulfate surfactant compositions, the method utilising the presence of a significant amount of mid-chain branched alcohol and/or polyoxyalkylene alcohol in the sulfation reaction to significantly reduce the reaction temperature, thereby improving product quality and saving energy. WO9404640A relates to a concentrated aqueous surfactant solution comprising alkyl ether sulfate and alkaline earth metal, preferably magnesium, the composition is a stable liquid which is suitable for making into cleaning products, especially dish washing liquids, the concentrated surfactant solution can be prepared by partial neutralisation of the acid precursor with the hydroxide or oxide of the alkaline earth metal, followed by a further neutralisation with the hydroxide of an alkali metal or ammonium. WO9105764A relates to the sulfatisation of ethoxylised alkanols obtained by the reaction of ethylene oxide with alcohols with 8 to 22 C atoms in the presence of a hydrotalcite catalyst provides alkyl polyethoxy ether sulfates distinguished by a low dioxane content and extremely good coagulability using ordinary electrolytes. US20170158625A relates to a process for preparing an alcohol ether sulfate which comprises: (a) sulfating an alkoxylated alcohol; and (b) neutralizing the sulfated product of step (a) in the presence of a base and a co-solvent having a flash point of at least 60° C. GB977281A relates to surface active sulfates of alkyl ether alcohols, wherein the ether-alcohol may be made by (a) the reaction of olefins with ethylene glycol, (b) the reaction of olefins with ethylene halohydrins, followed by hydrolysis of the halogen-containing products, or (c) the reaction of secondary or tertiary alcohols with

ethylene oxide, and wherein the products are all sulfated with chlorosulfonic acid. EP3919594A1 relates to a liquid detergent composition suitable for washing dishes, fitting both in-sink as well as direct application habits, which provides reduced smearing when used in direct application dishwashing methods, while having good suds mileage especially under in-sink application habit, and good viscosity, the liquid detergent composition comprising a surfactant system, which comprises an alkyl sulfate anionic surfactant comprising C13 alkyl sulfate anionic surfactant, the C13 alkyl sulfate anionic surfactant comprising a specific fraction of 2-branched C13 alkyl sulfate anionic surfactant, with a specific distribution of the 2-branching.

## SUMMARY OF THE INVENTION

The present invention relates to a process for making a concentrated surfactant blend, wherein the concentrated surfactant blend comprises alkyl sulfated anionic surfactant and a buffering surfactant selected from the group consisting of: amphoteric surfactant, zwitterionic surfactant, and mixtures thereof, wherein the process comprises the following steps: providing an alkyl alcohol stream comprising at least one alkyl alcohol; a sulfation step, during which the at least one alkyl alcohol in the alkyl alcohol stream is sulfated to form an alkyl sulfuric acid stream comprising at least one alkyl sulfuric acid; providing a neutralising stream comprising at least one neutralising agent; a neutralisation step, during which the alkyl sulfuric acid stream and the neutralising stream are combined, in order to neutralise the alkyl sulfuric acid; wherein the buffering surfactant is added before or during the neutralising step, the buffering surfactant is added at a level to provide the resultant concentrated surfactant blend with a reserve alkalinity of greater than 0.02, when measured as a 10% by weight solution of the concentrated surfactant blend in demineralized water at 20°C; and the resultant concentrated surfactant blend has a pH of from 7.1 to 10, when measured as a 10% by weight solution of the concentrated surfactant blend in demineralized water at 20°C.

The present invention further relates to a concentrated surfactant blend comprising: from 30 % to 70 % by weight of the concentrated surfactant blend of alkyl sulfated anionic surfactant, wherein the alkyl sulfated anionic surfactant has an average degree of alkoxylation of less than 0.5; from 1.0 % to 25 % by weight of the concentrated surfactant blend of a buffering surfactant selected from the group consisting of: amphoteric surfactant, zwitterionic surfactant, and mixtures thereof; and wherein the concentrated surfactant blend has a reserve alkalinity of greater than 0.02, when measured as a 10% by weight solution of the concentrated surfactant blend in demineralized water at 20°C, using the method as described herein; and the resultant concentrated surfactant blend has a pH of from 7.1 to 10, when measured as a 10% by weight solution of the concentrated surfactant blend in demineralized water at 20°C.

## DETAILED DESCRIPTION OF THE INVENTION

It has been found that adding the buffering surfactant before or during the neutralisation step results in a more hydrolytically stable concentrated surfactant blend, and as such, the concentrated surfactant blend can be kept at a lower pH. Furthermore, when formulating such concentrated surfactant blends into detergent compositions, less acid has to be added in order to reach the desired pH. As such, the resultant liquid detergent composition comprises less salt, and is hence more phase stable, especially at lower temperatures, without the need for additional organic solvents. In addition, the desired viscosity can be achieved with little or no organic solvent and/or added structurants. The lower salt content in the finished product composition has also been found to facilitate finished product dissolution, resulting in a faster onset of suds creation during washing accordingly. Furthermore, since the concentrated surfactant blend can be kept at a lower pH, the formation of 1,4-dioxanes is reduced or eliminated, both during making and during storage.

While salt-based buffer systems can limit pH drift of the concentrated surfactant blend, they introduce additional salts into the final detergent composition, which has a detrimental effect on both low temperature stability and viscosity. In contrast, the buffering surfactants of use herein provide detersive and sudsing benefit in addition to buffering, while not having a detrimental effect on composition viscosity and physical product stability.

As used herein, articles such as "a" and "an" when used in a claim, are understood to mean one or more of what is claimed or described.

The term "comprising" as used herein means that steps and ingredients other than those specifically mentioned can be added. This term encompasses the terms "consisting of' and "consisting essentially of." The compositions of the present invention can comprise, consist of, and consist essentially of the essential elements and limitations of the invention described herein, as well as any of the additional or optional ingredients, components, steps, or limitations described herein.

The term "dishware" as used herein includes cookware and tableware made from, by non-limiting examples, ceramic, china, metal, glass, plastic (e.g., polyethylene, polypropylene, polystyrene, etc.) and wood.

The term "grease" or "greasy" as used herein means materials comprising at least in part (*i.e.*, at least 0.5 wt% by weight of the grease in the material) saturated and unsaturated fats and oils, preferably oils and fats derived from animal sources such as beef, pig and/or chicken.

The terms "include", "includes" and "including" are meant to be non-limiting.

The term "particulate soils" as used herein means inorganic and especially organic, solid soil particles, especially food particles, such as for non-limiting examples: finely divided elemental carbon, baked grease particle, and meat particles.

The term "sudsing profile" as used herein refers to the properties of a cleaning composition relating to suds character during the dishwashing process. The term "sudsing profile" of a cleaning composition includes initial suds volume generated upon dissolving and agitation, typically manual agitation, of the cleaning composition in the aqueous washing solution, and the retention of the suds during the dishwashing process. Preferably, hand dishwashing cleaning compositions characterized as having "good sudsing profile" tend to have high initial suds volume and/or sustained suds volume, particularly during a substantial portion of or for the entire manual dishwashing process. This is important as the consumer uses high suds as an indicator that enough cleaning composition has been dosed. Moreover, the consumer also uses the sustained suds volume as an indicator that enough active cleaning ingredients (e.g., surfactants) are present, even towards the end of the dishwashing process. The consumer usually renews the washing solution when the sudsing subsides. Thus, a low sudsing cleaning composition will tend to be replaced by the consumer more frequently than is necessary because of the low sudsing level.

It is understood that the test methods that are disclosed in the Test Methods Section of the present application must be used to determine the respective values of the parameters of Applicants' inventions as described and claimed herein.

All percentages are by weight of the total composition, as evident by the context, unless specifically stated otherwise. All ratios are weight ratios, unless specifically stated otherwise, and all measurements are made at 25°C, unless otherwise designated.

Process for making a concentrated surfactant blend:

The present process is used to make a concentrated surfactant blend. The concentrated surfactant blend comprises alkyl sulfated anionic surfactant and a buffering surfactant selected from the group consisting of: amphoteric surfactant, zwitterionic surfactant, and mixtures thereof.

The process comprises the following steps:

a) providing an alkyl alcohol stream comprising at least one alkyl alcohol;
b) a sulfation step, during which the at least one alkyl alcohol in the alkyl alcohol stream is sulfated to form an alkyl sulfuric acid stream comprising at least one alkyl sulfuric acid;
c) providing a neutralising stream comprising at least one neutralising agent;
d) a neutralisation step, during which the alkyl sulfuric acid stream and the neutralising stream are combined, in order to neutralise the alkyl sulfuric acid;
wherein the buffering surfactant is added before or during the neutralising step.

The buffering surfactant is added at a level to provide the resultant concentrated surfactant blend with a reserve alkalinity of greater than 0.02 when measured as a 10% by weight solution of the concentrated surfactant blend in demineralized water at 20°C, using the method as described herein, with the resultant concentrated surfactant blend having a pH of from 7.1 to 10, when measured as a 10% by weight solution of the concentrated surfactant blend in demineralized water at 20°C.

The buffering capacity is the ability to neutralize the pH and the resistance to change in it due to the small acidic or basic inputs or discharges. When a system is poorly buffered, the addition of even small amounts of an acid or a base will noticeably alter its pH, but when a system is well buffered, the same addition barely modifies its pH.

Reserve alkalinity is a measure often used industrially to indicate the amount of alkaline components present in the product. For some compositions, such as the concentrated surfactant blends disclosed herein, it is more important to know the reserve alkalinity of the blend rather than the buffer capacity, because the reserve alkalinity provides a measure of the capacity of the blend to neutralise any acid that is present or formed *in-situ*, and hence maintain an alkaline pH.

Alkyl alcohol stream

The processes described herein comprise a step whereby an alkyl alcohol stream is provided. The alkyl alcohol stream comprises at least one alkyl alcohol. The alkyl alcohol stream as such can comprise one alkyl alcohol or alternatively a blend of alkyl alcohols.

The mol average alkyl chain length of the alkyl alcohol or of the blend of alkyl alcohols can be from 8 to 18, preferably from 10 to 14, more preferably from 12 to 14, most preferably from 12 to 13 carbon atoms, in order to provide

a combination of improved sudsing and grease removal and enhanced speed of cleaning from the resultant alkyl sulfate surfactant.

The alkyl chain of the alkyl alcohol or of the blend of alkyl alcohols can have a mol fraction of C12 and C13 chains of at least 50%, preferably at least 65%, more preferably at least 80%, most preferably at least 90%. Suds mileage is particularly improved, especially in the presence of greasy soils, when the C13/C12 mol ratio of the alkyl chain in the alkyl alcohol or in the blend of alkyl alcohols used to make the alkyl sulfate surfactant is at least 57/43, preferably from 60/40 to 90/10, more preferably from 60/40 to 80/20, most preferably from 60/40 to 70/30, while not compromising suds mileage in the presence of particulate soils.

The relative molar amounts of C13 and C12 alkyl chains in the alkyl alcohol or in the blend of alkyl alcohols can be derived from the carbon chain length distribution in the alkyl chain of the alkyl alcohol. The carbon chain length distribution of the alkyl chains of the alkyl alcohols can be obtained from the technical data sheets from the suppliers for the constituent alkyl alcohol. Alternatively, the chain length distribution and average molecular weight of the alkyl alcohols, used to make the alkyl sulfated anionic surfactant, can also be determined by methods known in the art. Such methods include capillary gas chromatography with flame ionisation detection on medium polar capillary column, using hexane as the solvent.

The alkyl alcohol or blends of alkyl alcohol can be alkoxylated or free of alkoxylation. Where it is desired that the resultant concentrated surfactant blend comprises alkyl alkoxylated sulfate, the at least one alkyl alcohol in the alkyl alcohol stream is preferably alkoxylated before the sulfation step. The alkyl alcohol can be a blend of alkyl alcohols which are first blended together and then jointly alkoxylated to provide the desired average degree of alkoxylation. Alternatively, one or more of alcohols can first be alkoxylated, and then the alkoxylated alcohols or alcohol blends can be mixed together to achieve the desired average degree of alkoxylation. In the first case, a monomodal distribution of the alkoxylation is typically achieved, while in the second case, the alkoxylation distribution is typically multi-modal.

When alkoxylated, the alkyl alcohol can have an average degree of alkoxylation of less than 3.5, preferably from 0.3 to 2.0, more preferably from 0.5 to 0.9, in order for the resultant alkyl sulfated anionic surfactant to improve low temperature physical stability and improve suds mileage of the compositions of the present invention. However, the average degree of alkoxylation (especially ethoxylation) of the starting alkyl alcohol is preferably less than 0.5, preferably less than 0.1, and more preferably the starting alkyl alcohol is free of alkoxylation, since the resultant alkyl sulfate anionic surfactant provide improved grease cleaning. When alkoxylated, ethoxylation is preferred.

The average degree of alkoxylation is the mol average degree of alkoxylation (*i.e.*, mol average alkoxylation degree) of all the alkyl alcohol. Hence, when calculating the mol average alkoxylation degree, the mols of non-alkoxylated alkyl alcohols are included:

$$\text{Mol average alkoxylation degree} = (x1 * \text{alkoxylation degree of alkyl alcohol 1} + x2 * \text{alkoxylation degree of alkyl alcohol 2} + \ldots) / (x1 + x2 + \ldots)$$

wherein x1, x2, ... are the number of moles of each alkyl (or alkoxy) alcohol of the mixture and alkoxylation degree is the number of alkoxy groups in each alkyl alcohol.

Preferred alkyl alkoxy alcohols for use in making the alkyl sulfate surfactant are alkyl ethoxy alcohols

The performance can be affected by the width of the alkoxylation distribution of the resultant alkoxylated alkyl sulfate anionic surfactant, including grease cleaning, sudsing, low temperature stability and viscosity of the finished product. The alkoxylation distribution, including its broadness can be varied through the selection of catalyst and process conditions when making the alkoxylated alkyl alcohol.

If alkoxylation, such as ethoxylation, of the alkyl alcohol is desired, without wishing to be bound by theory, through tight control of processing conditions and feedstock material compositions, both during alkoxylation especially ethoxylation and sulfation steps, the amount of 1,4-dioxane by-product within alkoxylated especially ethoxylated alkyl sulfates can be reduced. Based on recent advances in technology, a further reduction of 1,4-dioxane by-product can be achieved by subsequent stripping, distillation, evaporation, centrifugation, microwave irradiation, molecular sieving or catalytic or enzymatic degradation steps. Processes to control 1,4-dioxane content within alkoxylated/ethoxylated alkyl sulfates have been described extensively in the art. Alternatively 1,4-dioxane level control within detergent formulations has also been described in the art through addition of 1,4-dioxane inhibitors to 1,4-dioxane comprising formulations, such as 5,6-dihydro-3-(4-morpholinyl)-1-[4-(2-oxo-1-piperidinyl)-phenyl]-2-(1-H)-pyridone, 3-α-hydroxy-7-oxo stereoisomer-mixtures of cholinic acid, 3-(N- methyl amino)-L-alanine, and mixtures thereof.

The alkyl alcohol has a weight average degree of branching of from 15% to 50%, preferably from 20% to 40%. The use of such branched alkyl alcohols can result in improved low temperature stability for compositions comprising the resultant alkyl sulfate surfactant, as well as providing the desired grease cleaning performance.

Through tight control of the C2-branching of the alkyl alcohols, the resultant alkyl sulfate surfactants have been

found to provide liquid detergent compositions with improved product stability, even at low temperatures, and provide higher finished product viscosities, without compromising on suds mileage and grease cleaning. Furthermore, by limiting the average degree of alkoxylation (especially ethoxylation) of the starting alkyl alcohol to less than 0.5, preferably less than 0.1, and more preferably being free of alkoxylation, the resultant liquid detergent composition can have a reduced viscosensitivity with variations in the starting alcohol used to make the alkyl sulfate surfactant.

Moreover, such compositions require less solvent in order to achieve good physical stability at low temperatures. Higher surfactant branching also provides faster initial suds generation, but typically less suds mileage. The weight average branching, described herein, has been found to improve low temperature stability, initial foam generation and suds longevity in liquid detergent compositions comprising alkyl sulfate surfactants formed from such alkyl alcohols.

As such, the branched alkyl alcohol used to make the alkyl sulfate surfactant can comprise C2-branched alkyl alcohol and non-C2-branched alkyl alcohol. The weight ratio of non-C2-branched alkyl alcohol to C2-branched alkyl alcohol can be greater than 0.5, preferably from 1.0:1 to 5:1, more preferably from 2:1 to 4:1.

C2-branched means the alkyl branching is a single alkyl branching on the alkyl chain of the alkyl alcohol and is positioned on the C2 position, as measured counting carbon atoms from the hydroxyl group for non-alkoxylated alkyl alcohol, or counting from the alkoxy-group furthest from the hydroxyl group for alkoxylated alkyl alcohols.

Non-C2 branching means the alkyl chain comprises branching at multiple carbon positions along the alkyl chain backbone, or a single branching group present on a branching position on the alkyl chain other than the C2 position.

The non-C2 branched alkyl alcohol can comprise less than 30%, preferably less than 20%, more preferably less than 10% by weight of the non-C2 branched alkyl alcohol of C1-branched alkyl alcohol, most preferably the non-C2 branched alkyl alcohol is free of C1-branched alkyl alcohol.

The non-C2 branched alkyl alcohol can comprise at least 50%, preferably from 60 to 90%, more preferably from 70 to 80% by weight of the non-C2 branched alkyl alcohol of isomers comprising a single branching at a branching position greater than the 2-position. That is, more than 2 carbons atoms away from the hydrophilic headgroup, as defined above. The non-C2 branched alkyl alcohol can comprise from 5% to 30%, preferably from 7% to 20%, more preferably from 10% to 15% by weight of the non-C2 branched alkyl alcohol of multi branched isomers. The non-C2 branched alkyl alcohol can comprise from 5% to 30%, preferably from 7% to 20%, more preferably from 10% to 15% by weight of non-C2 branched alkyl alcohol of cyclic isomers. If present, the acyclic branching groups can be selected from C1 to C5 alkyl groups, and mixtures thereof.

The weight average degree of branching for an alkyl alcohol mixture can be calculated using the following formula:

$$\text{Weight average degree of branching (\%)} = [(x1 * \text{wt\% branched alkyl alcohol 1 in alcohol 1} + x2 * \text{wt\% branched alkyl alcohol 2 in alcohol 2} + \ldots) / (x1 + x2 + \ldots)] * 100$$

wherein x1, x2, ... are the weight in grams of each alkyl alcohol in the total alkyl alcohol mixture of the alkyl alcohols which were used as starting material before (alkoxylation and) sulfation to produce the alkyl (alkoxy) sulfate surfactant. In the weight average degree of branching calculation, the weight of the alkyl alcohol which is not branched is included.

The weight average degree of branching and the distribution of branching can typically be obtained from the technical data sheet for the surfactant or constituent alkyl alcohol. Alternatively, the branching can also be determined through analytical methods known in the art, including capillary gas chromatography with flame ionisation detection on medium polar capillary column, using hexane as the solvent. The weight average degree of branching and the distribution of branching is based on the starting alkyl alcohol used to produce the alkyl sulfated anionic surfactant.

Suitable examples of commercially available alkyl alcohols include, those derived from alcohols sold under the Neodol® brand-name by Shell, or the Lial®, Isalchem®, and Safol® brand-names by Sasol, or some of the natural alcohols produced by The Procter & Gamble Chemicals company. The alcohols (and alkoxylated alcohols) can be blended in order to achieve the desired mol fraction of C12 and C13 chains and the desired C13/C12 ratio, based on the relative fractions of C13 and C12 within the starting alcohols (as well as the desired degree of alkoxylation), as obtained from the technical data sheets from the suppliers or from analysis using methods known in the art.

The alkyl alcohol can comprise alkyl chains which are essentially linear or even fully linear, which are blended with the branched alcohol to achieve the desired degree of branching. Preferred sources of naturally derived alkyl chains include palm kernel and coconut derived alkyl chains, with palm kernel derived alkyl chains being more preferred. The naturally derived alkyl chain can be fractionated in order to provide the desired average alkyl chain length, as well as to adjust the alkyl chain length distribution. The C12 to C14 fraction is often referred to as the mid cut fraction within the naturally derived alkyl chains. Alternatively, essentially linear alkyl chains can be synthetically derived using the Ziegler process, or a derivative thereof, a method for producing fatty alcohols from ethylene using an organoaluminium compound. The reaction produces linear primary alcohols with an even numbered carbon chain. Again, the C12-C14 alkyl fraction

is preferred and can be fractionated out of the total Ziegler alcohol.

Sulfation

In the sulfation step, the alkyl alcohol stream comprising the at least one alkyl alcohol is sulfated to form an alkyl sulfuric acid stream comprising at least one alkyl sulfuric acid. Sulfation involves forming a carbon-oxygen-sulfur bond. The resultant alkyl sulfate in acid form (alkyl sulfuric acid) is not hydrolytically stable. Unless neutralized, it decomposes to form sulfuric acid and other chemicals.

Sulfation of alcohols or alkoxylated alcohols into (alkoxylated) alkyl sulfuric acids has been described extensively. More details of such a sulfation step has been described in "Sulf(on)ation Technology in the Detergent Industry" (W. Herman de Groot, Springer-Science+Business Media, B.V., 1991, ISBN 978-90-481-4088-6).

Various reagents can be used for the sulfation step, with sulfur trioxide ($SO_3$) being particularly preferred, at least partially due to its low cost. $SO_3$ is an aggressive electrophilic reagent that rapidly reacts with any organic compound containing an electron donor group. The resultant reaction is highly exothermic. Effective cooling of the reaction mass is essential because high temperatures promote side reactions that produce undesirable by-products. Also, precise control of the molar ratio of $SO_3$ to alkyl alcohol is essential because any excess $SO_3$, due to its reactive nature, contributes to side reactions and by-product formation. Therefore, commercial scale sulfation reactions require special equipment and instrumentation that allows tight control of the mole ratio of $SO_3$ to alkyl alcohol and rapid removal of the heat of reaction.

The problem of $SO_3$ reactivity has typically been solved by diluting and/or complexing the $SO_3$ to moderate the rate of reaction. Commercial diluting or complexing agents include ammonia (sulfamic acid), hydrochloric acid (chlorosulfuric acid), and dry air (air/$SO_3$ film sulfation). Control of the ratio of $SO_3$ to alkyl alcohol can be used to achieve improved product quality with use of any of these reagents.

Air/$SO_3$ film sulfation processes are typically carried out using a film reactor, such as an annular falling film reactor, such as a "Chemithon" reactor, or a multi-tube film reactor, such as the "Ballestra" reactor. In such processes, the $SO_3$ is first diluted with dry air. Such air/$SO_3$ sulfation processes are direct processes in which $SO_3$ gas is diluted with very dry air and reacted directly with the alkyl alcohol feedstock. The reaction of gaseous $SO_3$ with the alkyl alcohol is rapid and stoichiometric. Such processes are complicated by the possibility of side reactions, However, with tight process control, very high purity alkyl sulfate surfactants can be achieved.

The $SO_3$ can be provided by burning molten sulfur in an excess of oxygen to form $SO_2$, which is then catalytically oxidised to $SO_3$, for instance, at a temperature of from 400 °C to 470 °C. Conversion of sulfur to $SO_2$ is typically at least 95%, preferably at least 99%, more preferably at least >99.9% complete. The oxygen can be supplied by air which has been pre-dried to remove the major part of water by condensation and subsequently drying with a desiccant until the air has a maximum dewpoint of -60°C, preferably < -70°C. Considering the exothermic nature of the sulfur burning reaction, the $SO_2$/air flow is cooled in an indirect air cooler, prior to converting the $SO_2$ to $SO_3$ through a catalytic oxidation. Catalytic oxidation is typically done using at least one, preferably from 3 to 4 catalytic beds. An example of such a catalytic converter includes a converter tower filled with 4 packed beds of $V_2O_5$ catalyst on a silica carrier. Considering the exothermic nature of the $SO_2$ to $SO_3$ oxidation, an intermediate cooling of the resulting process gas is executed between the various beds through indirect air coolers, such as vertical air cooled shell and tube heat exchangers. Despite the air pre-drying step there is still some sulfuric acid / oleum mist condensed which is removed through a demister, such as a Brinks filter, prior to the sulfation step. To ensure sufficient mist is removed the gas stream at this point in the process is cooled to at least 60°C, but most preferably the gas stream is cooled to a temperature of from 30 to 55 °C.

In air/$SO_3$ film sulfation processes, the sulfation step is typically carried out in a liquid-gas interface reactor, preferably a falling film reactor. Suitable falling film reactors include annular-gap falling film ("Chemithon") reactors, (multi)tubular ("Ballestra") reactors, and the like.

The alkyl alcohol or blend of alkyl alcohols is converted through reaction with the $SO_3$ within the falling film reactor into alkyl sulfuric acid. Considering the exothermic nature of the sulfonation reaction, consequent cooling is again required after which the air/gas is separated from the liquid stream. Ideally the reaction mixture is maintained at the outlet of the reactor at a temperature of from 15 °C to 50 °C, preferably from 30 °C and 40°C.

The air/gas can be separated from the liquid using a liquid separator. Where the separation does not remove sufficient gas from the liquid mixture, an extra degassing step can be done, for instance, by using a "Fryma" spinning disc deaerator.

The exhaust gas typically comprises small amounts of non-converted $SO_2$, non-reacted $SO_3$ and some entrained organic acid. The organic aerosol and fine $SO_3$/$H_2SO_4$ droplets are typically separated from the exhaust gas flow in an electrostatic precipitator, and the gaseous $SO_2$ gasses are typically washed from the process air in a scrubber using a dilute caustic solution.

In order to minimise the formation of sulfuric acid and maintain a low ionic strength, the alkyl alcohol stream fed into the sulfation reaction preferably comprises less than 0.1%, preferably less than 0.05%, more preferably is free

of water.

A sulfation reaction typically results in a conversion of at least 90%, preferably at least 95%, more preferably at least 97% by weight of the starting alkyl alcohol. The sulfuric acid presence in the liquid stream after sulfation is typically less than 1%, preferably less than 0.75%, more preferably less than 0.5% by weight of the total liquid stream.

Neutralisation

Considering the sensitivity of protonated alkyl sulfuric acid to re-hydrolyse into the starting alkyl alcohols, a consequent neutralisation step is required. Without neutralisation, re-hydrolysis of the alkyl sulfuric acid leads to a decreased alkyl sulfated anionic surfactant content in the resultant concentrated surfactant blend, and also to an increased sulfuric acid content which results in discoloration of the concentrated surfactant blend and liquid detergent compositions made with the concentrated surfactant blend.

In the present process, a neutralising stream comprising at least one neutralising agent is provided. During or after the neutralisation step, the neutralising agent can be added at a level to provide the resultant concentrated surfactant blend with a pH of from 7.1 to 10, preferably from 7.3 to 9.5, more preferably from 7.5 to 9.0, measured as a 10% by weight solution of the concentrated surfactant blend in demineralized water, at 20°C.

Too low a pH can lead to re-hydrolysis of the alkyl sulfated anionic surfactant, while too high a pH typically complicates conversion of the concentrated surfactant blend into a finished detergent product composition, such as requiring a significant amount of acid to trim back the pH to the targeted finished product pH. In addition, too high a pH can result in the generation of 1,4-dioxanes over time, as well as higher amounts of neutralizing agent being added, increasing the salt level in the concentrated surfactant blend accordingly. Concentrated surfactant blends which comprise high salt levels are more challenging to formulate into detergent compositions, and result in a reduced low temperature stability as well as increased viscosity upon initial dissolution with water, slowing down product dissolution accordingly.

The neutralising agent is an alkali. Further neutralising agent can be added after the neutralisation step in order to adjust the pH to the desired level. Suitable alkali can be selected from the group consisting of sodium hydroxide potassium hydroxide, ammonia, mono-ethanolamine, di-ethanolamine, tri-ethanolamine, and mixtures thereof, with sodium hydroxide being most preferred. Alternatively, or in addition, other alkalis can be added as part of the neutralisation step. Particularly suitable other alkalis are alkalis that can be added to the resultant liquid detergent composition to improve performance. In particular, amines, especially cyclic polyamine having amine functionalities that helps cleaning, as described later.

The neutralisation step should be completed in less than 10 minutes, preferably less than 5 minutes and most preferably less than 2 minutes, after the sulfation step has been completed.

The neutralisation can take place in any suitable means, including in a batch reactor or by combining the alkyl sulfuric acid stream and neutralising stream using a high shear mixer. In preferred processes, a loop reactor can be used. A loop reactor is a continuous tube or pipe, typically stainless steel, which connects the outlet of a recirculation pump to its inlet. Reactants are fed into the loop where the neutralisation occurs, and the at least partially neutralised mixture is withdrawn from the loop.

As such, a loop reactor typically comprises a circulation pump, a homogenizer or high shear mixer, and a heat exchanger, due to the exothermic nature of the neutralisation reaction. Efficient mixing of the alkyl sulfuric acid stream and the neutralising stream results in instantaneous reaction and avoids undesired degradation reactions in isolated spots and pH drift occurring during the neutralisation step. As such high shear mixers are typically used considering the highly viscous nature of the resulting paste, especially at low shear rates.

As the neutralization reaction is exothermic, a heat exchanger, such as a plate and frame heat exchanger, can be used to continuously cool the mixture to achieve a temperature of less than 70°C, preferably less than 60°C and most preferably in a range of 20 to 40°C for the alkyl sulfate stream after neutralisation.

Water or organic solvents can be added either to control the viscosity during the neutralisation step or to improve homogenisation. Suitable organic solvents include C1-C4 alcohols, particularly ethanol. Such organic solvents can be added as a separate stream during the neutralisation step, or as part of the alkyl alcohol stream, or as part of the neutralisation stream. Water can be added as a separate stream during the neutralisation step, or as part of the neutralisation stream.

Water can be added at a level such that the resultant concentrated surfactant blend comprises water at a level of from 20% to 50%, preferably from 25% to 45%, more preferably from 30% to 40% by weight of the concentrated surfactant blend. The water level can be measured using any suitable means, such as via Karl Fisher Titration,

The neutralisation stream can comprise water at a level of from 35% to 80%, preferably from 45% to 75%, more preferably from 55% to 65% by weight of the neutralisation stream.

The buffering surfactant is preferably added during the neutralisation step to ensure that the resulting mixture reaches the desired pH quickly so as to avoid the aforementioned hydrolysis.

The neutralising stream can comprise other optional ingredients, such as nonionic surfactant, polymers, and

peroxides, such as those described later, and mixtures thereof.

After the neutralization step the resulting neutralized surfactant paste can be transported through a transfer pump into a storage tank.

Buffering surfactant:

The buffering surfactant is preferably added during the neutralization step, either as a separate buffer stream, but is preferably added as part of the neutralisation stream. Alternatively, the buffering surfactant can be added before the neutralisation step. The alkyl sulfuric acid and the buffering surfactant can be combined in a weight ratio of the alkyl sulfuric acid to the buffering surfactant of from 10:1 to 1:1, preferably from 8:1 to 2:1, more preferably from 6:1 to 3:1.

The buffering surfactant reduces the pH variation and pH drift when small amounts of acid are added or formed, for instance, through hydrolysis of the alkyl sulfate surfactant, or even from absorption of carbon dioxide from the air. As a result, the resultant concentrated surfactant blend can be stored at lower pH, resulting in less formation of 1,4-dioxanes and less salt being present in detergent compositions comprising the concentrated surfactant blend.

The buffering surfactant is an amphoteric surfactant, zwitterionic surfactant, or mixtures thereof. Suitable buffering surfactants can be selected from the group consisting of: amine oxide surfactant, betaine surfactant, and mixtures thereof, preferably amine oxide surfactant, more preferably C10-16 dimethyl amine oxide surfactant. C12-14 dimethyl amine oxide (lauryl dimethyl amine oxide) is particularly preferred. Such buffering surfactants also contribute to the performance of the resultant liquid hand dishwashing detergent. In addition, the buffering surfactant results in the concentrated surfactant blends, formed by the processes described herein, having reduced viscosity, especially when amine oxide surfactant is used as the buffering surfactant.

The buffering surfactant can be added at a level such that the resultant concentrated surfactant blend comprises the buffering surfactant at a level of from 1.0% to 25% preferably from 5.0% to 20%, more preferably from 10% to 15% by weight of the concentrated surfactant blend.

Suitable amine oxide surfactants can be linear or branched, though linear are preferred. Suitable linear amine oxides are typically water-soluble, and characterized by the formula R1-N(R2)(R3) O wherein R1 is a C8-18 alkyl, and the R2 and R3 moieties are selected from the group consisting of C1-3 alkyl groups, C1-3 hydroxyalkyl groups, and mixtures thereof. For instance, R2 and R3 can be selected from the group consisting of: methyl, ethyl, propyl, isopropyl, 2-hydroxethyl, 2-hydroxypropyl and 3-hydroxypropyl, and mixtures thereof, though methyl is preferred for one or both of R2 and R3. The linear amine oxide surfactants in particular may include linear C10-C18 alkyl dimethyl amine oxides and linear C8-C12 alkoxy ethyl dihydroxy ethyl amine oxides.

Preferably, the amine oxide surfactant is selected from the group consisting of: alkyl dimethyl amine oxide, alkyl amido propyl dimethyl amine oxide, and mixtures thereof. Alkyl dimethyl amine oxides are particularly preferred, such as C8-18 alkyl dimethyl amine oxides, or C10-16 alkyl dimethyl amine oxides (such as coco dimethyl amine oxide). Suitable alkyl dimethyl amine oxides include C10 alkyl dimethyl amine oxide surfactant, C10-12 alkyl dimethyl amine oxide surfactant, C12-C14 alkyl dimethyl amine oxide surfactant, and mixtures thereof. C12-C14 alkyl dimethyl amine oxide, C12-14 alkyl amido propyl amine oxide, and mixtures thereof are particularly preferred.

Alternative suitable amine oxide surfactants include mid-branched amine oxide surfactants. As used herein, "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of n1 and n2 can be from 10 to 24 carbon atoms, preferably from 12 to 20, and more preferably from 10 to 16. The number of carbon atoms for the one alkyl moiety (n1) is preferably the same or similar to the number of carbon atoms as the one alkyl branch (n2) such that the one alkyl moiety and the one alkyl branch are symmetric. As used herein "symmetric" means that | n1 - n2 | is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties, independently selected from a CI-3 alkyl, a CI-3 hydroxyalkyl group, or a polyethylene oxide group containing an average of from about 1 to about 3 ethylene oxide groups. Preferably, the two moieties are selected from a CI-3 alkyl, more preferably both are selected as C1 alkyl.

Alternatively, the amine oxide surfactant can be a mixture of amine oxides comprising a mixture of low-cut amine oxide and mid-cut amine oxide. The amine oxide of the composition of the invention can then comprises:

a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls and mixtures thereof; and

b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl, with preferably both R1 and R2 being methyl. In the mid-cut amine oxide of formula R4R5R6AO, R4 and R5 are preferably both methyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Limiting the amount of amine oxides of formula R7R8R9AO improves both physical stability and suds mileage.

Suitable zwitterionic surfactants include betaine surfactants. Such betaine surfactants includes alkyl betaines, alkylamidobetaine, amidazoliniumbetaine, sulfobetaine (INCI Sultaines) as well as the phosphobetaine, and preferably meets formula (I):

$$R^1\text{-}[CO\text{-}X(CH_2)_n]_x\text{-}N^+(R^2)(R_3)\text{-}(CH_2)_m\text{-}[CH(OH)\text{-}CH_2]_y\text{-}Y^-$$

Wherein in formula (I),

R1 is selected from the group consisting of: a saturated or unsaturated C6-22 alkyl residue, preferably C8-18 alkyl residue, more preferably a saturated C10-16 alkyl residue, most preferably a saturated C12-14 alkyl residue;
X is selected from the group consisting of: NH, NR4 wherein R4 is a C1-4 alkyl residue, O, and S,
n is an integer from 1 to 10, preferably 2 to 5, more preferably 3,
x is 0 or 1, preferably 1,
R2 and R3 are independently selected from the group consisting of: a C1-4 alkyl residue, hydroxy substituted such as a hydroxyethyl, and mixtures thereof, preferably both R2 and R3 are methyl,
m is an integer from 1 to 4, preferably 1, 2 or 3,
y is 0 or 1, and
Y is selected from the group consisting of: COO, SO3, OPO(OR5)O or P(O)(OR5)O, wherein R5 is H or a C1-4 alkyl residue.

Preferred betaines are the alkyl betaines of formula (Ia), the alkyl amido propyl betaine of formula (Ib), the sulfobetaine of formula (Ic) and the amido sulfobetaine of formula (Id):

$$R^1\text{-}N^+(CH_3)_2\text{-}CH_2COO^- \qquad \text{(Ia)}$$

$$R^1\text{-}CO\text{-}NH\text{-}(CH_2)_3\text{-}N^+(CH_3)_2CH_2COO^- \qquad \text{(Ib)}$$

$$R^1\text{-}N^+(CH_3)_2CH_2CH(OH)CH_2SO_3^- \qquad \text{(Ic)}$$

$$R^1\text{-}CO\text{-}NH\text{-}(CH_2)_3\text{-}N^+(CH_3)_2CH_2CH(OH)CH_2SO_3^- \qquad \text{(Id)}$$

in which R1 has the same meaning as in formula (I). Particularly preferred are the carbobetaines [i.e. wherein Y-=COO- in formula (I)] of formulae (Ia) and (Ib), more preferred are the alkylamidobetaine of formula (Ib).

Suitable betaines can be selected from the group consisting or [designated in accordance with INCI]: capryl/capramidopropyl betaine, cetyl betaine, cetyl amidopropyl betaine, cocamidoethyl betaine, cocamidopropyl betaine, cocobetaines, decyl betaine, decyl amidopropyl betaine, hydrogenated tallow betaine / amidopropyl betaine, isostearamidopropyl betaine, lauramidopropyl betaine, lauryl betaine, myristyl amidopropyl betaine, myristyl betaine, oleamidopropyl betaine, oleyl betaine, palmamidopropyl betaine, palmitamidopropyl betaine, palm-kernelamidopropyl betaine, stearamidopropyl betaine, stearyl betaine, tallowamidopropyl betaine, tallow betaine, undecylenamidopropyl betaine, undecyl betaine, and mixtures thereof. Preferred betaines are selected from the group consisting of: cocamidopropyl betaine, cocobetaines, lauramidopropyl betaine, lauryl betaine, myristyl amidopropyl betaine, myristyl betaine, and mixtures thereof. Cocamidopropyl betaine is particularly preferred.

In the neutralisation step, the at least one alkyl sulfuric acid and the buffering surfactant can be combined in a weight ratio of from 10:1 to 1:1, preferably from 8:1 to 2:1, more preferably from 6:1 to 3:1.

As such, the unreacted alkyl alcohol and/or alkoxylated alcohol level is preferably below 3%, more preferably below 2.5%, and most preferably below 2% by weight of the starting alkyl sulfuric acid at the start of the neutralisation step. The amount of unreacted alkyl (alkoxylated) alcohol can be determined by GC analysis (after neutralisation of the alkyl sulfuric acid).

The buffering surfactant can be added in a stream which further comprises peroxide, especially where amine oxide is used as a buffering surfactant. The peroxide can be present such that the stream comprising the buffering surfactant has a residual peroxide level of from 5.0 ppm to 300 ppm, preferably from 40 ppm to 80 ppm for every one part by weight of buffering surfactant. It is believed that the presence of peroxide further limits the formation and growth

of 1,4-dioxane within the concentrated surfactant blend formed by the present process, when the concentrated surfactant blend comprises ethoxylated alkyl sulfate surfactant.

Optional further steps

If desired, adjunct ingredients as described below can also be added prior to, during or after neutralisation, for instance, to simplify making of the final liquid detergent composition. Other optional ingredients include water, organic solvents, pH modifier, further surfactants, polymers, amines, preservatives, and mixtures thereof. Suitable further surfactants include further anionic surfactants, nonionic surfactants, and mixtures thereof.

Alkyl sulfate containing concentrated surfactant blend

The resultant concentrated surfactant blend comprises: from 30 wt% to 70 wt%, preferably from 40 wt% to 60 wt%, more preferably from 45 wt% to 55 wt% by weight of the resultant concentrated surfactant blend of the alkyl sulfated anionic surfactant, and from 1.0 wt% to 25 wt%, preferably from 5.0 wt% to 20 wt%, more preferably from 10 wt% to 15 wt% by weight of the resultant concentrated surfactant blend of the buffering surfactant. The concentrated surfactant blend comprises alkyl sulfated anionic surfactant, wherein the alkyl sulfated anionic surfactant has an average degree of alkoxylation of less than 0.5, preferably less than 0.1, more preferably is free of alkoxylation. When alkoxylated, the alkoxylation preferably is ethoxylation.

The buffering surfactant buffers the pH of the concentrated surfactant blend without requiring additional salts to be introduced into the blend. As such, the concentrated surfactant blend resists changes in pH due to the addition or formation of an acid (or base). The resultant buffering reduces the risk of hydrolysis of the alkyl sulfate surfactant due to lowering pH, for example, upon ageing in contact with air, lowering the alkyl sulfate concentration but also resulting in the formation of by-products which cause browning of the concentrated surfactant blend (such as $HSO_4^-$). Moreover, without the buffering, a higher pH would be required for the concentrated surfactant blend, in order to avoid the pH dropping over time to a level that hydrolysis of the alkyl sulfate surfactant occurs too rapidly. The need to add additional alkali results in a higher ionic strength for unbuffered concentrated surfactant blends. This leads to reduced processability of the concentrated surfactant blend, as well as reduced dispersibility upon dilution, lower viscosity, as well as reduced foaming and suds milage for compositions comprising such unbuffered concentrated surfactant blends.

Buffers for a given application must be effective at the desired pH, and must also provide sufficient buffer capacity to maintain the desired pH as needed. In order to inhibit hydrolysis and minimise the formation of 1,4-dioxanes, the resultant concentrated surfactant blend, formed by the processes described herein, preferably have a buffer region of from a pH of 7.0 to 7.8.

A measure of the efficacy of the buffering system is provided by the reserve alkalinity. The reserve alkalinity is essentially the ability of the composition to neutralise acidic residues that are formed in the composition, such as by the hydrolysis of the alkyl sulfate surfactant. The buffering surfactant is added at a level to provide the resultant concentrated surfactant blend with a reserve alkalinity of greater than 0.02, preferably from 0.04 to 0.50, more preferably from 0.06 to 0.30 when measured as a 10% by weight solution of the concentrated surfactant blend in demineralized water at 20°C, using the method described herein.

The alkyl sulfated anionic surfactant and the buffering surfactant can be present in the concentrated surfactant blend in a weight ratio of from 10:1 to 1:1, preferably from 8:1 to 2:1, more preferably from 6:1 to 3:1.

It has been found that the present process, utilising the buffering surfactant, enables a lower pH for the concentrated surfactant blend. As a result, lower levels of 1,4-dioxane are present in the concentrated surfactant blend. Moreover, the resultant alkyl sulfate containing concentrated surfactant blends exhibit lower rates of increase in the 1,4-dioxane by-product level upon ageing. As such, the dioxane level in the concentrated surfactant blends formed by the process of the present invention can be less than 40ppm, preferably less than 30, most preferably less than 15ppm.

The concentrated surfactant blend can comprise further surfactants. Suitable further surfactants include further anionic surfactants such as sulfonate anionic surfactants such as HLAS, or sulfosuccinate anionic surfactants. However, in preferred processes, the amount of such further anionic surfactants is kept low. In more preferred processes, no further anionic surfactant is added. Therefore, the concentrated surfactant blend can comprise at least 70%, preferably at least 85%, more preferably 100% by weight of the anionic surfactant of the alkyl sulfated anionic surfactant. The concentrated surfactant blend is preferably free of fatty acid or salt thereof, since such fatty acids impede the generation of suds.

Suitable further surfactants include nonionic surfactants. The concentrated surfactant blend can further comprise a nonionic surfactant. Suitable nonionic surfactants include alkoxylated alcohol nonionic surfactants, alkyl polyglucoside nonionic surfactants, and mixtures thereof.

The concentrated surfactant blend can comprise from 1% to 25%, preferably from 1.25% to 20%, more preferably from 1.5% to 15%, most preferably from 1.5% to 5%, by weight of the total surfactant within the concentrated surfactant

blend, of an alkoxylated alcohol non-ionic surfactant.

Preferably, the alkoxylated alcohol non-ionic surfactant is a linear or branched, primary or secondary alkyl alkoxylated non-ionic surfactant, preferably an alkyl ethoxylated non-ionic surfactant, preferably comprising on average from 9 to 15, preferably from 10 to 14 carbon atoms in its alkyl chain and on average from 5 to 12, preferably from 6 to 10, most preferably from 7 to 8, units of ethylene oxide per mole of alcohol.

The concentrated surfactant blend can comprise alkyl polyglucoside ("APG") surfactant. The addition of alkyl polyglucoside surfactants has been found to improve sudsing beyond that of comparative nonionic surfactants such as alkyl ethoxylated nonionic surfactants. If present, the alkyl polyglucoside can be present in the concentrated surfactant blend at a level of from 0.5% to 20%, preferably from 0.75% to 15%, more preferably from 1% to 10%, most preferably from 1% to 5% by weight of the total surfactant within the concentrated surfactant blend. Preferably the alkyl polyglucoside surfactant is a C8-C16 alkyl polyglucoside surfactant, preferably a C8-C14 alkyl polyglucoside surfactant. The alkyl polyglucoside preferably has an average degree of polymerization of between 0.1 and 3, more preferably between 0.5 and 2.5, even more preferably between 1 and 2. Most preferably, the alkyl polyglucoside surfactant has an average alkyl carbon chain length between 10 and 16, preferably between 10 and 14, most preferably between 12 and 14, with an average degree of polymerization of between 0.5 and 2.5 preferably between 1 and 2, most preferably between 1.2 and 1.6.

C8-C16 alkyl polyglucosides are commercially available from several suppliers (e.g., Simusol® surfactants from Seppic Corporation; and Glucopon® 600 CSUP, Glucopon® 650 EC, Glucopon® 600 CSUP/MB, and Glucopon® 650 EC/MB, from BASF Corporation).

The resultant concentrated surfactant blend can have a pH of from 7.1 to 10, preferably from 7.3 to 9.5, more preferably from 7.5 to 9.0, when measured as a 10% by weight solution of the concentrated surfactant blend in demineralized water at 20 °C. Too low a pH can lead to re-hydrolysis of the alkyl sulfate, while too high a pH typically complicates conversion of the concentrated surfactant blend into a finished detergent product composition, such as requiring a significant amount of acid to trim back the pH to the targeted finished product pH. In addition, too high a pH can result in the generation of 1,4-dioxanes over time, as well as needing higher amounts of neutralizing agent to be added, increasing the salt level in the concentrated surfactant blend accordingly. Concentrated surfactant blends which comprise high salt levels are more challenging to formulate into detergent compositions, and result in a reduced low temperature stability as well as increased viscosity upon initial dissolution with water, slowing down product dissolution.

The concentrated surfactant blend can be Newtonian or non-Newtonian, preferably Newtonian. The surfactant blend can have a viscosity of from 5,000 mPa·s to 25,000 mPa·s, preferably from 7,500 mPa·s to 20,000 mPa·s, most preferably from 10,000 mPa·s to 15,000 mPa·s, measured at a shear rate of 10 $s^{-1}$ and a temperature of 20° C.

The concentrated surfactant blend can have a flow index of from 0.1 to 1.0, preferably from 0.2 to 0.5, more preferably from 0.2 to 0.4. The concentrated surfactant blend can have a yield stress of from 5.0 Pa to 30 Pa, preferably from 10 Pa to 25 Pa, more preferably from 15 Pa to 20 Pa. The aforementioned flow index and yield stress result in improved processibility of the concentrated surfactant blend.

The melting point of the concentrated surfactant blend is preferably less than 20 °C, more preferably less than 15 °C, most preferably less than 10 °C so that the concentrated surfactant blend can be stored at ambient temperatures and not require heating in order to process into a detergent composition. The melting point can be measured using Differential Scanning Calorimetry" (DSC).

The concentrated surfactant blend can have a density in the range from 500 $kg/m^3$ to 1,500 $kg/m^3$, preferably from 750 $kg/m^3$ to 1,250 $kg/m^3$, more preferably from 1,000 $kg/m^3$ to 1,100 $kg/m^3$, measured at a temperature of 20 °C. The density can be measured using any suitable means, such as using a pycnometer.

<u>Liquid hand dishwashing composition:</u>

The concentrated surfactant blends, as described herein can be used to make liquid detergent compositions, especially liquid hand dishwashing detergent composition.

The term "dishware" and "dish" as used herein includes cookware and tableware made from, by non-limiting examples, ceramic, china, metal, glass, plastic (e.g., polyethylene, polypropylene, polystyrene, etc.) and wood.

The cleaning composition is a liquid cleaning composition, preferably a liquid hand dishwashing cleaning composition, and hence is in liquid form. The liquid cleaning composition is preferably an aqueous cleaning composition. As such, the composition can comprise from 50% to 85%, preferably from 50% to 75%, by weight of the total composition of water.

The liquid cleaning composition has a pH greater than 6.0, or a pH of from 6.0 to 12.0, preferably from 7.0 to 11.0, more preferably from 8.0 to 10.0, measured as a 10% aqueous solution in demineralized water at 20 degrees °C.

When the pH exceeds a pH of 7.0, the reserve alkalinity can be from 0.1 to 1.0, more preferably from 0.1 to 0.5. Reserve alkalinity is herein expressed as grams of NaOH/100 ml of composition required to titrate product from a pH 7.0 to the pH of the finished composition. This pH and reserve alkalinity further contribute to the cleaning of tough

food soils.

The liquid cleaning composition of the present invention can be Newtonian or non-Newtonian, preferably Newtonian. Preferably, the composition has a viscosity of from 10 mPa·s to 10,000 mPa·s, preferably from 100 mPa·s to 5,000 mPa·s, more preferably from 300 mPa·s to 2,000 mPa·s, or most preferably from 500 mPa·s to 1,500 mPa·s, alternatively combinations thereof. The viscosity is measured at 20°C with a Brookfield RT Viscometer using spindle 31 with the RPM of the viscometer adjusted to achieve a torque of between 40% and 60%.

The liquid cleaning composition can comprise the concentrated surfactant blend and optionally additional surfactant, such that the composition comprises from 5.0% to 50%, preferably from 6.0% to 40%, most preferably from 15% to 35%, by weight of the total composition of a surfactant system.

The liquid hand dishwashing detergent composition comprises anionic surfactant that is comprised in the concentrated surfactant blend used to make the detergent composition. Preferably, the liquid hand dishwashing detergent composition does not comprise any further anionic surfactant.

The liquid hand dishwashing detergent composition comprises a co-surfactant which comprises or consists of the buffering surfactant comprised in the concentrated surfactant blend used to make the detergent composition. Further co-surfactant can be added, in addition to the co-surfactant comprised in the concentrated surfactant blend. The liquid hand dishwashing detergent composition can comprise nonionic surfactant. Suitable nonionic surfactants include alkoxylated alcohol nonionic surfactants, alkyl polyglucoside nonionic surfactants, and mixtures thereof, as described earlier. Such nonionic surfactant can be added as part of the concentrated surfactant blend used to make the detergent composition, or can be added separately and in addition to the concentrated surfactant blend, or both.

The composition can comprise further ingredients such as those selected from: amphiphilic alkoxylated polyalkyleneimines, cyclic polyamines, triblock copolymers, inorganic mono-, di- or trivalent salts, hydrotropes, organic solvents, other adjunct ingredients such as those described herein, and mixtures thereof. Such ingredients can be added as part of the surfactant blend, or separately added, in addition to the surfactant blend.

Amphiphilic alkoxylated polyalkyleneimine:

The composition of the present invention may further comprise from 0.05% to 2%, preferably from 0.07% to 1% by weight of the total composition of an amphiphilic polymer. Suitable amphiphilic polymers can be selected from the group consisting of: amphiphilic alkoxylated polyalkyleneimine and mixtures thereof. The amphiphilic alkoxylated polyalkyleneimine polymer has been found to improve grease removal.

A preferred amphiphilic alkoxylated polyethyleneimine polymer has the general structure of formula (I):

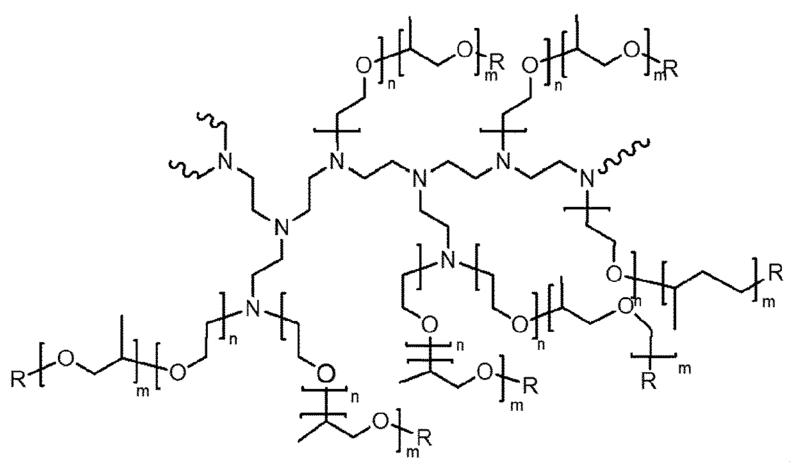

(I)

wherein the polyethyleneimine backbone has a weight average molecular weight of 600, n of formula (I) has an average of 10, m of formula (I) has an average of 7 and R of formula (I) is selected from hydrogen, a $C_1$-$C_4$ alkyl and mixtures thereof, preferably hydrogen. The degree of permanent quaternization of formula (I) may be from 0% to 22% of the polyethyleneimine backbone nitrogen atoms. The molecular weight of this amphiphilic alkoxylated polyethyleneimine polymer preferably is between 10,000 and 15,000 Da.

More preferably, the amphiphilic alkoxylated polyethyleneimine polymer has the general structure of formula (I) but wherein the polyethyleneimine backbone has a weight average molecular weight of 600 Da, n of Formula (I) has an average of 24, m of Formula (I) has an average of 16 and R of Formula (I) is selected from hydrogen, a $C_1$-$C_4$ alkyl and mixtures thereof, preferably hydrogen. The degree of permanent quaternization of Formula (I) may be from 0% to 22% of the polyethyleneimine backbone nitrogen atoms and is preferably 0%. The molecular weight of this amphiphilic alkoxylated polyethyleneimine polymer preferably is between 25,000 and 30,000, most preferably 28,000 Da.

The amphiphilic alkoxylated polyethyleneimine polymers can be made by the methods described in more detail in PCT Publication No. WO 2007/135645.

Cyclic Polyamine

The composition can comprise a cyclic polyamine having amine functionalities that helps cleaning. The composition of the invention preferably comprises from 0.1% to 3%, more preferably from 0.2% to 2%, and especially from 0.5% to 1%, by weight of the composition, of the cyclic polyamine.
The cyclic polyamine has at least two primary amine functionalities. The primary amines can be in any position in the cyclic amine but it has been found that in terms of grease cleaning, better performance is obtained when the primary amines are in positions 1,3. It has also been found that cyclic amines in which one of the substituents is -CH3 and the rest are H provided for improved grease cleaning performance.
Accordingly, the most preferred cyclic polyamine for use with the cleaning composition of the present invention are cyclic polyamine selected from the group consisting of: 2-methylcyclohexane-1,3-diamine, 4-methylcyclohexane-1,3-diamine and mixtures thereof. These specific cyclic polyamines work to improve suds and grease cleaning profile through-out the dishwashing process when formulated together with the surfactant system of the composition of the present invention.
Suitable cyclic polyamines can be supplied by BASF, under the Baxxodur tradename, with Baxxodur ECX-210 being particularly preferred.
A combination of the cyclic polyamine and magnesium sulfate is particularly preferred. As such, the composition can further comprise magnesium sulfate at a level of from 0.001 % to 2.0 %, preferably from 0.005 % to 1.0 %, more preferably from 0.01 % to 0.5 % by weight of the composition.

Triblock Copolymer

The composition of the invention can comprise a triblock copolymer. The triblock co-polymers can be present at a level of from 0.1% to 10%, preferably from 0.5% to 7.5%, more preferably from 1% to 5%, by weight of the total composition. Suitable triblock copolymers include alkylene oxide triblock co-polymers, defined as a triblock co-polymer having alkylene oxide moieties according to Formula (I): (EO)x(PO)y(EO)x, wherein EO represents ethylene oxide, and each x represents the number of EO units within the EO block. Each x can independently be on average of from 5 to 50, preferably from 10 to 40, more preferably from 10 to 30. Preferably x is the same for both EO blocks, wherein the "same" means that the x between the two EO blocks varies within a maximum 2 units, preferably within a maximum of 1 unit, more preferably both x's are the same number of units. PO represents propylene oxide, and y represents the number of PO units in the PO block. Each y can on average be from between 28 to 60, preferably from 30 to 55, more preferably from 30 to 48.
Preferably the triblock co-polymer has a ratio of y to each x of from 3:1 to 2:1. The triblock co-polymer preferably has a ratio of y to the average x of 2 EO blocks of from 3:1 to 2:1. Preferably the triblock co-polymer has an average weight percentage of total EO of between 30% and 50% by weight of the tri-block co-polymer. Preferably the triblock co-polymer has an average weight percentage of total PO of between 50% and 70% by weight of the triblock co-polymer. It is understood that the average total weight % of EO and PO for the triblock co-polymer adds up to 100%. The triblock co-polymer can have an average molecular weight of between 2060 and 7880, preferably between 2620 and 6710, more preferably between 2620 and 5430, most preferably between 2800 and 4700. Average molecular weight is determined using a 1H NMR spectroscopy (*see* Thermo scientific application note No. AN52907).
Triblock co-polymers have the basic structure ABA, wherein A and B are different homopolymeric and/or monomeric units. In this case A is ethylene oxide (EO) and B is propylene oxide (PO). Those skilled in the art will recognize the phrase "block copolymers" is synonymous with this definition of "block polymers".
Triblock co-polymers according to Formula (I) with the specific EO/PO/EO arrangement and respective homopolymeric lengths have been found to enhance suds mileage performance of the liquid hand dishwashing detergent composition in the presence of greasy soils and/or suds consistency throughout dilution in the wash process.
Suitable EO-PO-EO triblock co-polymers are commercially available from BASF such as Pluronic® PE series, and from the Dow Chemical Company such as Tergitol™ L series. Particularly preferred triblock co-polymer from BASF are sold under the tradenames Pluronic® PE6400 (MW ca 2900, ca 40wt% EO) and Pluronic® PE 9400 (MW ca 4600, 40 wt% EO). Particularly preferred triblock co-polymer from the Dow Chemical Company is sold under the tradename Tergitol™ L64 (MW ca 2700, ca 40 wt% EO).
Preferred triblock co-polymers are readily biodegradable under aerobic conditions.
The composition of the present invention may further comprise at least one active selected from the group consisting of: salt, hydrotrope, organic solvent, and mixtures thereof.

Salt:

The composition of the present invention may comprise from 0.05% to 2%, preferably from 0.1% to 1.5%, or more preferably from 0.5% to 1%, by weight of the total composition of a salt, preferably a monovalent or divalent inorganic salt, or a mixture thereof, more preferably selected from: sodium chloride, sodium sulfate, and mixtures thereof. Sodium chloride is most preferred.

Hydrotrope:

The composition of the present invention may comprise from 0.1% to 10%, or preferably from 0.5% to 10%, or more preferably from 1% to 10% by weight of the total composition of a hydrotrope or a mixture thereof, preferably sodium cumene sulfonate.

Organic Solvent:

The composition can comprise from 0.1% to 10%, or preferably from 0.5% to 10%, or more preferably from 1% to 10% by weight of the total composition of an organic solvent. Suitable organic solvents include organic solvents selected from the group consisting of: alcohols, glycols, glycol ethers, and mixtures thereof, preferably alcohols, glycols, and mixtures thereof. Ethanol is the preferred alcohol. Polyalkyleneglycols, especially polypropyleneglycol (PPG), are the preferred glycol. The polypropyleneglycol can have a molecular weight of from 400 to 3000, preferably from 600 to 1500, more preferably from 700 to 1300. The polypropyleneglycol is preferably poly-1,2-propyleneglycol.

Adjunct Ingredients

The cleaning composition may optionally comprise a number of other adjunct ingredients such as builders (preferably citrate), further chelants, conditioning polymers, other cleaning polymers, surface modifying polymers, structurants, emollients, humectants, skin rejuvenating actives, enzymes, carboxylic acids, scrubbing particles, perfumes, malodor control agents, pigments, dyes, opacifiers, pearlescent particles, inorganic cations such as alkaline earth metals such as Ca/Mg-ions, antibacterial agents, preservatives, antioxidants, viscosity adjusters (e.g., salt such as NaCl, and other mono-, di- and trivalent salts) and pH adjusters and buffering means (e.g. carboxylic acids such as citric acid, HCl, NaOH, KOH, alkanolamines, carbonates such as sodium carbonates, bicarbonates, sesquicarbonates, and alike).

If present, the composition comprises from 0.01% to 2.0%, preferably from 0.05% to 1.5%, or more preferably from 0.1% to 1.0%, by weight of alkaline earth metal ions, with magnesium and/or calcium ions being particularly preferred. Low levels of transition metal ions can also be present in the liquid detergent composition, such as up to 1.0%, or up to 0.5% by weight of the composition.

Packaged product

The hand dishwashing detergent composition can be packaged in a container, typically plastic containers. Suitable containers comprise an orifice. Typically, the container comprises a cap, with the orifice typically comprised on the cap. The cap can comprise a spout, with the orifice at the exit of the spout. The spout can have a length of from 0.5 mm to 10 mm.

The orifice can have an open cross-sectional surface area at the exit of from 3 $mm^2$ to 20 $mm^2$, preferably from 3.8 $mm^2$ to 12 $mm^2$, more preferably from 5 $mm^2$ to 10 $mm^2$, wherein the container further comprises the composition according to the invention. The cross-sectional surface area is measured perpendicular to the liquid exit from the container (that is, perpendicular to the liquid flow during dispensing).

The container can typically comprise from 200 ml to 5,000 ml, preferably from 350 ml to 2000 ml, more preferably from 400 ml to 1,000 ml of the liquid hand dishwashing detergent composition.

Alternatively, the hand dishwashing detergent composition can be packaged in an inverted container. Such inverted containers typically comprise a cap at the bottom of the container, the cap comprising either a closure or a self-sealing valve, or a combination thereof. The cap preferably comprises a self-sealing valve. Suitable self-sealing valves include slit-valves. The self-sealing valve defines a dispensing orifice that is reactably openable when the pressure on the valve interior side exceeds the pressure on the valve exterior side. The bottom dispensing container can comprise an impact resistance system, such as that described in WO2019108293A1.

Method of Washing

The resultant liquid detergent compositions can be used for manually washing. Suitable methods can comprise

the steps of delivering such a liquid detergent composition to a volume of water to form a wash solution and immersing the dishware in the solution. The dishware is be cleaned with the composition in the presence of water. The dishware can be rinsed. By "rinsing", it is meant herein contacting the dishware cleaned with the process according to the present invention with substantial quantities of appropriate solvent, typically water. By "substantial quantities", it is meant usually about 1 to about 20 L, or under running water.

The composition herein can be applied in its diluted form. Soiled dishware are contacted with an effective amount, typically from about 0.5 mL to about 20 mL (per about 25 dishes being treated), preferably from about 3 mL to about 10 mL, of the cleaning composition, preferably in liquid form, of the present invention diluted in water. The actual amount of cleaning composition used will be based on the judgment of the user and will typically depend upon factors such as the particular product formulation of the cleaning composition, including the concentration of active ingredients in the cleaning composition, the number of soiled dishes to be cleaned, the degree of soiling on the dishes, and the like. Generally, from about 0.01 mL to about 150 mL, preferably from about 3 mL to about 40 mL of a cleaning composition of the invention is combined with from about 2,000 mL to about 20,000 mL, more typically from about 5,000 mL to about 15,000 mL of water in a sink. The soiled dishware is immersed in the sink containing the diluted cleaning compositions then obtained, before contacting the soiled surface of the dishware with a cloth, sponge, or similar cleaning implement. The cloth, sponge, or similar cleaning implement may be immersed in the cleaning composition and water mixture prior to being contacted with the dishware, and is typically contacted with the dishware for a period of time ranged from about 1 to about 10 seconds, although the actual time will vary with each application and user. The contacting of cloth, sponge, or similar cleaning implement to the dishware is accompanied by a concurrent scrubbing of the dishware.

Preferably, the composition is applied in its neat form to the dish to be treated. By "in its neat form", it is meant herein that said composition is applied directly onto the surface to be treated, or onto a cleaning device or implement such as a brush, a sponge, a nonwoven material, or a woven material, without undergoing any significant dilution by the user (immediately) prior to application. Application using a sponge is preferred. "In its neat form", also includes slight dilutions, for instance, arising from the presence of water on the cleaning device, or the addition of water by the consumer to remove the remaining quantities of the composition from a bottle. Therefore, the composition in its neat form includes mixtures having the composition and water at ratios ranging from 50:50 to 100:0, preferably 70:30 to 100:0, more preferably 80:20 to 100:0, even more preferably 90:10 to 100:0 depending on the user habits and the cleaning task.

## TEST METHODS

### A) pH:

The pH is measured as a 10% aqueous solution in demineralized water at 20 °C, using a pH meter, such as an Orion Model 720A with an Ag/AgCl electrode (for example an Orion sure flow Electrode model 9172BN), calibrated using standardized pH 7 and pH 10 buffers.

### B) Reserve alkalinity:

Reserve alkalinity is defined as the grams of NaOH per 100 g of composition required to titrate the test composition at pH 7.0 to come to the test composition pH. The reserve alkalinity for a solution is determined in the following manner.

The reserve alkalinity is measured at a 10% solution of the concentrated surfactant blend in deionized water at 20°C. As such, 50g of the concentrated surfactant blend is diluted to 10% with deionized water and mixed for 5 minutes until fully homogenized. 100g of the 10% solution is then titrated using an automated titrator, such as the Omnis sample robot and Omnis titrator, supplied by Metrohm, using 0.1 N hydrochloric acid (HCl) and pH meter (as above), until an endpoint of pH 4 is achieved or the maximum titration amount of the titrator is reached (25 ml for the titrator above). The volume of the titrant required to reach a pH of 7.0 is recorded, or if the maximum titration amount is reached, the final pH and volume of titrant is recorded.

The reserve alkalinity is calculated as follows:

$$\text{Reserve Alkalinity} = \text{ml } 0.1\text{N HCl required to reach a pH of } 7.0 \times 0.1 \text{ (equivalent / liter)} \times \text{Equivalent weight NaOH (g/equivalent)} \times 10.$$

Where the maximum titration amount from the titrator has been reached, the final pH is recorded and the reserve alkalinity to this pH is calculated. The reserve alkalinity to pH 7.0 can then be estimated through linear extrapolation to pH 7.0 of the obtained "pH versus volume titrant" titration curves between an added titrant volume of 20ml and 25 ml.

C) Concentrated surfactant blend viscosity:

The rheology profile is measured using a "TA instruments DHR1" rheometer, using a cone and plate geometry with a flat steel Peltier plate and a 40 mm diameter, 2.008° cone (TA instruments, serial number: SN999393). The viscosity measurement procedure includes a conditioning step and a sweep step at 20 °C. The conditioning step takes place at 20°C and consists of 10 seconds at zero shear, followed by pre-shearing for 10 seconds at 10 $s^{-1}$, followed by 30 seconds at zero shear in order for the sample to equilibrate.

An upwards shear rate sweep is conducted from a shear rate of from 0.1$s^{-1}$ to 100$s^{-1}$, in increments of 10 points per decade, each incremental step is executed by the rheometer automatically after stabilisation of the measurement. Unless otherwise stated, the viscosity is measured at shear rate of 10 $s^{-1}$ and a temperature of 20 °C.

D) Flow index and yield stress of the concentrated surfactant blend:

As with the viscosity measurements of the concentrated surfactant blends, the rheology profile is measured using a "TA instruments DHR1" rheometer, using a cone and plate geometry with a flat steel Peltier plate and a 40 mm diameter, 2.008° cone (TA instruments, serial number: SN999393). The viscosity measurement procedure includes a conditioning step and a sweep step at 20 °C. The conditioning step takes place at 20°C and consists of 10 seconds at zero shear, followed by pre-shearing for 10 seconds at 10 $s^{-1}$, followed by 30 seconds at zero shear in order for the sample to equilibrate.

An upwards shear rate sweep is conducted from a shear rate of from 0.1$s^{-1}$ to 100$s^{-1}$, in increments of 10 points per decade, each incremental step is executed by the rheometer automatically after stabilisation of the measurement.

The shear stress is recorded as a function of shear rate by the rheometer. The data is then fitted to the Herschley Buckley model: $\tau = \tau_0 + K \gamma^n$, where "$\tau$" is the shear stress, "$\tau_0$" is the yield stress, and is y the shear rate. "K" is the consistency index, and "n" is the flow index.

D) Concentrated surfactant blend viscosity variation with temperature:

The rheology variation with temperature is measured using a "TA instruments DHR1" rheometer, using a cone and plate geometry with a flat steel Peltier plate and a 40 mm diameter, 2.026° cone (TA instruments, serial number: SN999393), at a shear rate of 10$s^{-1}$ and a temperature sweep of from 60°C down to 5°C using a temperature ramp of 2°C/min, after a 100 second equilibration step at a shear rate of 10$s^{-1}$ at 60°C.

E) Detergent composition viscosity measurement:

The viscosity of a detergent composition is measured at 20°C with a Brookfield RT Viscometer using spindle 31 with the RPM of the viscometer adjusted to achieve a torque of between 40% and 60%.

F) Foamability of detergent composition:

The initial foamability of a composition is assessed using a KRUSS DFA100 Dynamic Foam Analyzer. The detergent composition is diluted to 50% in water having a hardness of 2.67 mmol/l equivalent of $CaCO_3$ at 22 °C. 50 ml of the diluted solution is dosed using a syringe into a standard glass column (CY4501) fitted onto the Quick Fit Unit for agitation (SH4512), equipped with the standard agitator blade (SR4501). The initial solution height is recorded.
The solution is then agitated for 5 seconds at 5000 RPM. 20 seconds after agitation has stopped, the total sample height is recorded (foam + liquid solution). To calculate the height of the foam, the initial solution height is distracted from the total height measured.

G) Suds mileage index:

The objective of the Suds Mileage Test is to compare the evolution over time of suds volume generated for different test formulations at specified water hardness, solution temperatures and formulation concentrations, while under the influence of periodic soil injections. Data are compared and expressed versus a reference composition as a suds mileage index (reference composition has suds mileage index of 100). The steps of the method are as follows:

1. 0.12 wt% of the test composition is dispensed through a plastic pipette at a flow rate of 0.67 mL/ sec at a height of 37 cm above the bottom surface of a sink (dimension: 300 mm diameter and 288 mm height) into water streams having a water hardness of 0.36 mmol/L equivalence of calcium carbonate (2.0 gpg), 1.25 mmol/L equivalence of calcium carbonate (7.0 gpg), and 2.67 mmol/L equivalence of calcium carbonate (15 gpg) and water temperature

of 35°C, that is filling up the sink to 4 L at a constant pressure of 4 bar.

2. An initial suds volume generated (measured as average foam volume X above the liquid in the sink (expressed in $cm^3$) is recorded immediately after end of filling.
3. A fixed amount (6 mL) of a soil with the defined composition below is immediately injected into the middle of the sink.
4. The resultant solution is mixed with a metal blade (10 cm x 5 cm) positioned in the middle of the sink at the air liquid interface under an angle of 45° rotating at 85 RPM for 20 revolutions.
5. Another measurement of the total suds volume is recorded immediately after end of blade rotation.
6. Steps 3-5 are repeated until the measured total suds volume reaches a level of 400 $cm^3$ or less. The amount of added soil that is needed to get to the 400 $cm^3$ level is considered as the suds mileage for the test composition.
7. Each test composition is tested 4 times per testing condition (i.e., water temperature, composition concentration, water hardness, soil type).
8. The average suds mileage is calculated as the average of the 4 replicates for each sample for a defined test condition.
9. The Suds Mileage Index is calculated by comparing the average mileage of a test composition sample versus a reference composition sample. The calculation is as follows:

$$\text{Suds Mileage Index} = \frac{\text{Average number of soil additions of test composition}}{\text{Average number of soil additions of reference composition}} \text{ X } 100$$

The greasy soil composition used in the test is produced through standard mixing of the components described in Table A.

Table A: Greasy Soil

| Ingredient | Weight % |
|---|---|
| Crisco Oil | 12.730 |
| Crisco shortening | 27.752 |
| Lard | 7.638 |
| Refined Rendered Edible Beef Tallow | 51.684 |
| Oleic Acid, 90% (Techn) | 0.139 |
| Palmitic Acid, 99+% | 0.036 |
| Stearic Acid, 99+% | 0.021 |

EXAMPLES

The effect of alkyl sulfuric acid neutralization in presence of amine oxide buffering surfactant (inventive) and absence of amine oxide buffering surfactant (comparative) on the rheology of the resultant concentrated surfactant blend, as well as on the finished product dilution, foaming and physical stability has been studied following the test methods described herein. The buffered concentrated surfactant blends were made on the pilot plant scale using SO3 gas to sulfate the material in a falling film reactor and neutralized as described herein.

For the inventive examples, the neutralising stream was an aqueous solution of sodium hydroxide and C12-C14 dimethyl amine oxide (as the buffering surfactant), at the levels given in table 1. For the comparative examples, the neutralising stream was an aqueous solution of sodium hydroxide, with no buffering surfactant added.

The blend of example 1 (of the invention) comprised branched alkyl sulfuric acid having a weight average degree of branching of 50% and amine oxide buffering surfactant at a ratio of 4.4:1. The blend of comparative example A was similar to example 1 but comprised a weight average degree of branching of 55% (above that required by the present invention). The blend of comparative example B was similar to example 1, except that a linear alkyl sulfuric acid was used.

Comparative blend examples D to E used the same alkyl sulfuric acid as inventive example 1 and comparative examples A and B, respectively but did not comprise buffering surfactant. As such, a higher level of sodium hydroxide was needed to ensure the pH was sufficiently high to achieve a robust reserve alkalinity (to avoid re-hydrolysis of the alkyl sulfuric acid).

Table 1: levels of actives present during the neutralisation step for the inventive concentrated surfactant blends.

| Concentrated surfactant blend | Ex 1 | Ex A* | Ex B* | Ex C* | Ex D* | Ex E* |
|---|---|---|---|---|---|---|
| C12-C13 branched alkyl sulfuric acid[1] | 51.1 | - | - | 69.7 | - | - |
| C12-C13 branched alkyl sulfuric acid[2] | - | 49.8 | - | - | 68.1 | - |
| C12-14 linear alkyl branched alkyl sulfuric acid [3] | - | - | 51.3 | - | - | 66.7 |
| Alkali agent (NaOH) | 6.5 | 6.5 | 6.3 | 10.5 | 10.25 | 9.3 |
| Buffering surfactant[4] | 11.7 | 12.0 | 11.6 | - | - | - |
| Water | to 100% | to 100% | to 100% | to 100% | to 100% | to 100% |
| | | | | | | |
| Molar ratio alkyl sulfuric acid : Buffering surfactant | 4.4:1 | 4.4:1 | 4.4:1 | - | - | - |
| pH (trimmed using NaOH ) | 7.8 | 8.0 | 8.1 | 11.7 | 11.7 | n.m. |
| Reserve alkalinity | 0.073 | 0.094 | 0.071 | 0.059 | 0.040 | n.m. |
| Viscosity (Pa.s) at 5°C | 12.1 | 2.1 | 72.5 | 50.3 | gel | gel |
| 8°C | 8.6 | 1.8 | 60.8 | 28.3 | gel | gel |
| 16°C | 2.3 | 1.4 | 17.0 | 10.2 | gel | gel |
| 22°C | 1.7 | 1.3 | 6.7 | 11.3 | gel | gel |
| 27 °C | 1.6 | 1.2 | 3.2 | 7.1 | gel | gel |
| 30 °C | 1.6 | 1.2 | 2.6 | 5.4 | gel | gel |

* Comparative
[1] 50% branched C12-C13 alkyl sulfate, produced from Safol™ 23 alcohol, a Fischer-Tropsh derived alcohol, supplied by Sasol
[2] 55% branched C12-C13 alkyl sulfate, produced from Lial™ 123 alcohol, an OXO alcohol, supplied by Sasol
[3] Linear C12-C14 alkyl sulfate, produced by P&G Chemicals, from naturally derived alkyl chains distilled from palm kernel oil
[4] C12-C14 dimethylamine oxide, produced by P&G Chemicals

As can be seen from the results in Table 1a above, the compositions of the present invention, comprising the buffering surfactant, had a higher reserve alkalinity and hence greater robustness against rehydrolysis of the alkyl sulfuric acid, even though less alkali (sodium hydroxide) was used. In addition, the lower amounts of alkali resulted in less salt being present in the concentrated surfactant blend, resulting in a more stable and pourable viscosity.

Moreover, as can be seen from the viscosity data, the concentrated surfactant blends of the present invention, comprising the buffering surfactant, have a more readily processible viscosity profile, in contrast to the comparative compositions which do not comprise the buffering surfactant. The small difference in surfactant level between the inventive and comparative examples does not give rise to the sharp difference in viscosity that has been measured.

Indeed, the concentrated surfactant blends of comparative compositions C and D had a viscosity that was an almost solid gel, such that the viscosity was not even measurable. Adding water to these compositions in order to arrive at the same active level as inventive compositions 1 and comparative compositions A and B resulted in composition C entering the gel phase and composition D remaining in the highly viscous gel-phase, with the viscosity being not measurable for both.

Example 2 is a further example of the present invention, and examples F to H are further comparative examples (having a degree of branching outside the present invention) using different ratios of anionic surfactant to buffering surfactant.

Table 2: levels of actives present during the neutralisation step for the different concentrated surfactant blends.

| Concentrated surfactant blend | Ex 2 | Ex F* | Ex G* | Ex. H* |
|---|---|---|---|---|
| C12-C13 branched alkyl sulfuric acid[1] | - | - | - | - |
| C12-C13 branched alkyl sulfuric acid [2] | 42.0 | 36.4 | 30.8 | - |
| C12-14 linear alkyl branched alkyl sulfuric acid [3] | - | - | - | 51.7 |
| Alkali agent (NaOH) | 5.5 | 4.8 | 4.0 | 6.2 |
| Buffering surfactant[4] | 20.0 | 25.7 | 31.4 | 11.7 |
| Water | to 100% | to 100% | to 100% | To 100% |
| | | | | |
| Molar ratio alkyl sulfuric acid : Buffering surfactant | 3:1 | 2:1 | 1:1 | 4.4:1 |
| pH (trimmed using NaOH ) | 8.6 | 8.9 | 9.1 | 8.1 |
| Reserve alkalinity | 0.201 | 0.246[5] | 0.262[5] | n.m. |
| [5] extrapolated value (titration limit reached) | | | | |

The surfactant blend of comparative example B (see table 1 above) was remade (comparative example H) and formulated into a detergent composition (comparative detergent example B). Comparative detergent example A was formulated using a commercial concentrated alkyl sulfate blend not comprising a buffering surfactant (Tensopol® S30LSHPH, commercially available from KLK Oleo). Tensopol® S30LSHPH is a 30% active sodium lauryl sulfate in liquid form, having a high pH (greater than pH 11.0). The pH of detergent composition B was adjusted (using sodium hydroxide) to arrive at a pH of 9.2. The pH of comparative detergent composition A was adjusted (using citric acid) to arrive at the same pH of 9.2. The resultant compositions are given below in table 3. The amine oxide level given for detergent composition B includes a part added with the concentrated surfactant blend of comparative example H, and a part that was subsequently added when making the composition. Since it is not known how much alkali was present in the Tensopol® S30LSHPH starting material for detergent composition A, this is marked as "unknown".

Table 3: Liquid hand dishwashing detergent compositions

| Wt% (100% active basis) | Detergent composition A* | Detergent composition B* |
|---|---|---|
| Sodium alkyl sulfate | 15.11 | 15.11 |
| C12-14 dimethyl amine oxide | 5.40 | 5.40 |
| C8-10 APG (Glucopon 215UP) | 1.75 | 1.75 |
| C10-16 APG (Glucopon 600 CSUP) | 1.75 | 1.75 |
| Lauryl betaine (Euroquat CF) | 1.0 | 1.0 |
| NaOH | unknown | 0.2 |
| Citric acid | 0.31 | - |
| ethanol | 4.0 | 4.0 |
| 1,2-PPG (MW 1000) | 1.2 | 1.2 |
| Water and minors (perfume, dye, preservative) | to 100% | to 100% |
| | | |
| pH (measured as 10% solution at 20°C) | 9.2 | 9.2 |
| | | |
| Viscosity (cps) | 693 | 759 |

In order to assess the dispersibility of the detergent compositions, detergent composition B (made using comparative surfactant blend H which was made using the process of the invention but using alcohol having linear alkyl chains) and detergent composition A (comparative and made using a different process) were diluted using demineralized water to the concentrations shown in table 3 below and the viscosities measured using the Brookfield viscosity test method described herein. The lower the viscosity upon dilution, the more readily the composition disperses as it is added to a sink full of water.

Table 4: % of initial viscosity as the detergent compositions are diluted

| % remaining viscosity | Detergent composition A* | Detergent composition B |
|---|---|---|
| 100% product concentration | 100 | 100 |
| 90% product concentration | 90 | 64 |
| 80% product concentration | 47 | 36 |
| 70% product concentration | 22 | 13 |
| 60% product concentration | 7 | 4 |
| 50% product concentration | 2 | 1 |

As can be seen from the data above, the compositions produced from concentrated surfactant blends made using the process of the present invention are more readily dispersed - even though they comprise the same level of active ingredients and had essentially the same starting viscosity. While the above comparative detergent composition B was made using a comparative surfactant blend having a degree of branching above that required in the present invention (55%), the improvement in viscosity upon dilution can be interpolated for surfactant blends made with the present process, using alkyl alcohol having the degree of branching required for the present invention.

In addition, the foamability of comparative detergent composition B and comparative detergent composition A were assessed. The foamability was measured for the two compositions following the foamability test method described herein. The amount of foam generated was summarized in table 4.

Table 5: Foamability of the detergent compositions at 50% product concentration

| | Detergent composition B* | Detergent composition A* |
|---|---|---|
| foam height (mm) | 82 | 72 |

As can be seen from the data above, the compositions produced from the concentrated surfactant blends made using the present process, albite comprising anionic surfactant having a degree of branching above that required by the present invention, are more readily foaming - even though they comprise the same level of active ingredients. Again, while the above comparative detergent composition B was made using a comparative surfactant blend having a degree of branching above that required in the present invention (55%), the improvement in foamability can be extrapolated to surfactant blends made with the present process, using alkyl alcohol having the degree of branching required for the present invention.

The surfactant blend of comparative example H (made with the present process, but using an alkyl alcohol having a weight average degree of branching of 55%, see table 3 above) was formulated to form comparative detergent composition C. Comparative detergent example D was formulated using the same commercial concentrated alkyl sulfate blend as used to make comparative detergent composition A (Tensopol®S30LSHPH, commercially available from KLK Oleo). In both cases, the pH was adjusted to pH 9.2. The resultant detergent compositions are given in table 6 below.

Table 6: Liquid hand dishwashing detergent compositions

| Wt% (100% active basis) | Detergent composition C* | Detergent composition D* |
|---|---|---|
| Sodium alkyl sulfate | 15.84 | 15.84 |
| C12-14 dimethyl amine oxide | 5.66 | 5.66 |
| C8-10 APG (Glucopon 215UP) | 2.75 | 2.75 |
| C10-16 APG (Glucopon 600 CSUP) | 2.75 | 2.75 |

(continued)

| Wt% (100% active basis) | Detergent composition C* | Detergent composition D* |
|---|---|---|
| NaOH | 0.2 | unknown |
| Citric acid | - | 0.31 |
| ethanol | 4.0 | 4.0 |
| 1,2-PPG (MW 1000) | 1.2 | 1.3 |
| Water and minors (perfume, dye, preservative) | to 100% | to 100% |
| Viscosity | | |
| pH (measured as 10% solution at 20°C) | 9.2 | 9.2 |
| | | |
| Viscosity (cps) | 779 | 867 |
| foam height (mm) (50% concentration) | 79 | 71 |
| Freeze thaw recovery | Hazy & lumps after freezing, full recovery at room temperature | Hazy & lumps after freezing, thick bottom layer remaining upon recovery at room temperature |
| Foam mileage index** | 100 | 101 |
| averaged across 2, 7 and 15 gpg water hardness | | |

As can be seen from the data above, the compositions produced with the process of the invention, but using alkyl alcohol having a weight average degree of branching of 55% (detergent composition C) are more readily foaming than equivalent detergent compositions made using surfactant blends formed from traditional processes - even though they comprise the same level of active ingredients, and have improved freeze-thaw recovery, without negatively impacting foam mileage in the presence of greasy soils. While the above comparative detergent composition C was made using a comparative surfactant blend having a degree of branching above that required in the present invention (55%), the improvement in low temperature stability can be interpolated for surfactant blends made with the present process, using alkyl alcohol having the degree of branching required for the present invention.

The effect of branching of the alkyl alcohol in the present process is demonstrated by the comparative test below: Three alcohol blends were used: Alcohol blend 1 had a degree of branching of 42.1%. Alcohol blend A had a higher degree of branching of 62.5% (again primarily C2 branching), while alcohol blend B had a higher degree of branching of 86.6%. Alcohol blends 1 (of use in the invention) and A and B were primarily branched at the C2 position.

All the blends comprised the same C13 branched (non-ethoxylated) alcohol, C13 branched ethoxylated (to degree 3.0) alcohol and C12-14 linear (non-ethoxylated) alcohol, mixed together at different ratios to achieve an average degree of ethoxylation of 0.6 and the desired degree of branching.

The sulfation step was done on a pilot plant scale using SO3 gas to sulfate the material in a falling film reactor.

C12-C14 dimethyl amine oxide was added as the buffering surfactant during the neutralisation step, to provide a weight ratio of the alkyl sulfuric acid to the buffering surfactant of 4.4:1.

The sulfation reaction completion rate was measured for each alcohol blend.

Table 7: Alkyl chain distribution of starting C13 alcohols, and resultant completion rate for the sulfation reaction.

| | Alcohol blend 1 | Alcohol blend A | Alcohol blend B |
|---|---|---|---|
| Average alkyl chain length | C13 | C13 | C13 |
| Branched content+ | 42.1 | 62.5 | 86.6% |
| Ethoxylation degree | 0.6 | 0.6 | 0.6 |
| | | | |

(continued)

| | Alcohol blend 1 | Alcohol blend A | Alcohol blend B |
|---|---|---|---|
| Reaction completion | 98.7 | 97.3 | 95.06 |
| + by weight of starting C13 alcohol, the remainder being linear alcohol | | | |

As can be seen from the data above, in processes of the present invention, limiting the degree of branching of the starting alcohol as required by the present invention leads to an improvement in reaction completion rate. As a result, in addition to the improved reaction yield, less unreacted alcohol is present. The improvement in reaction yield leads to a more cost effective process. The reduction in unreacted alcohol provides the additional benefit of improved low temperature stability of both the concentrated surfactant blend and also of detergent compositions made with such blends. This is because unreacted alcohols in particular are more hydrophobic than the sulphated surfactants, and act as seeds for the precipitation of other ingredients, and hence result in reduced phase stability, especially at low temperatures.

It is known in the art that to increase the reaction completeness, higher levels of SO3 are needed. Hence, running at high completeness is economically beneficial, leads to a reduced level of unreacted alkyl alcohol, but in traditional processes, usually results in higher sulphuric acid levels in the acid blend after sulfation, since the molar ratio of SO3 to alcohol has to be increased above the stoichotric ratio. In traditional processes, this has typically meant that more alkali is needed in the neutralisation step, leading to higher salt levels in the resultant concentrated surfactant blends.

In contrast, in the processes of the present invention wherein a buffering surfactant is added before or during the neutralising step, this additional salt is at least reduced. As a result, the processes of the present invention result a higher reaction yield, less unreacted alkyl alcohol being present, and less salt being present. Both the reduction in unreacted alkyl alcohol and reduction in salt levels result in improved stability of the resultant concentrated surfactant blend and of detergent compositions made using them, especially at lower temperatures.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A process for making a concentrated surfactant blend, wherein the concentrated surfactant blend comprises alkyl sulfated anionic surfactant and a buffering surfactant selected from the group consisting of: amphoteric surfactant, zwitterionic surfactant, and mixtures thereof, wherein the process comprises the following steps:

a. providing an alkyl alcohol stream comprising at least one alkyl alcohol, wherein the alkyl alcohol in the alkyl alcohol stream has a weight average degree of branching of from 15% to 50%;
b. a sulfation step, during which the at least one alkyl alcohol in the alkyl alcohol stream is sulfated to form an alkyl sulfuric acid stream comprising at least one alkyl sulfuric acid;
c. providing a neutralising stream comprising at least one neutralising agent;
d. a neutralisation step, during which the alkyl sulfuric acid stream and the neutralising stream are combined, in order to neutralise the alkyl sulfuric acid;

wherein:

the buffering surfactant is added before or during the neutralising step;
the buffering surfactant is added at a level to provide the resulting concentrated surfactant blend with a reserve alkalinity of greater than 0.02 when measured as a 10% by weight solution of the concentrated surfactant blend in demineralized water at 20°C; and
the resultant concentrated surfactant blend has a pH of from 7.1 to 10 when measured as a 10% by weight solution of the concentrated surfactant blend in demineralized water at 20 °C.

**2.** The process according to claim 1, wherein the alkyl alcohol in the alkyl alcohol stream comprises a blend of alkyl alcohols.

**3.** The process according to any preceding claims, wherein the alkyl alcohol in the alkyl alcohol stream has a mol

average alkyl chain length of from 8 to 18, preferably from 10 to 14, most preferably from 12 to 13 carbon atoms.

**4.** The process according to any preceding claims, wherein the alkyl alcohol in the alkyl alcohol stream has a weight average degree of branching of from 20% to 40%.

**5.** The process according to any preceding claims, wherein the alkyl alcohol in the alkyl alcohol stream prior to the sulfation step has an average degree of alkoxylation of less than 0.5, preferably less than 0.1, more preferably is free of alkoxylation.

**6.** The process according to claim 5, wherein the alkyl alcohol comprises branched alkyl alcohol, wherein

a. the branched alkyl alcohol comprises C2-branched alkyl alcohol and non-C2-branched alkyl alcohol, wherein the weight ratio of non-C2-branched alkyl alcohol to C2-branched alkyl alcohol is greater than 0.5, preferably from 1.0:1 to 5:1, more preferably from 2:1 to 4:1; and
b. the non-C2 branched alkyl alcohol comprises less than 30%, preferably less than 20%, more preferably less than 10% by weight of the non-C2 branched alkyl alcohol of C1-branched alkyl alcohol, most preferably the non-C2 branched alkyl alcohol is free of C1-branched alkyl alcohol

**7.** The process according to any of claims 1 to 4, wherein the at least one alkyl alcohol in the alkyl alcohol stream is alkoxylated to an average degree of alkoxylation of less than 3.5, preferably from 0.3 to 2.0, more preferably from 0.5 to 0.9.

**8.** The process according to any preceding claims, wherein the sulfation step is carried out in a liquid-gas interface reactor, preferably a falling film reactor.

**9.** The process according to any preceding claims, wherein the buffering surfactant is added during the neutralisation step.

**10.** The process according to any preceding claims, wherein the buffering surfactant is selected from the group consisting of: amine oxide surfactant, betaine surfactant, and mixtures thereof, preferably amine oxide surfactant, more preferably C12-C14 alkyl dimethyl amine oxide, C12-14 alkyl amido propyl amine oxide, and mixtures thereof.

**11.** The process according to any preceding claims, wherein the alkyl sulfuric acid and the buffering surfactant are combined in a weight ratio of the alkyl sulfuric acid to the buffering surfactant of from 10:1 to 1:1, preferably from 8:1 to 2:1, more preferably from 6:1 to 3:1.

**12.** The process according to any preceding claims, wherein during or after the neutralisation step, the neutralising agent is added at a level to provide the resultant concentrated surfactant blend with a pH of from 7.3 to 9.5, more preferably from 7.5 to 9.0, measured as a 10% by weight solution of the concentrated surfactant blend in demineralized water, at 20°C.

**13.** The process according to any preceding claims, wherein the buffering surfactant is added at a level to provide the resultant concentrated surfactant blend with a reserve alkalinity of from 0.04 to 0.50, more preferably from 0.06 to 0.30, when measured as a 10% by weight solution of the concentrated surfactant blend in demineralized water at 20°C.

**14.** A concentrated surfactant blend comprising:

a. from 30 % to 70 % by weight of the concentrated surfactant blend of alkyl sulfated anionic surfactant, wherein the alkyl sulfated anionic surfactant has an average degree of alkoxylation of less than 0.5, and a weight average degree of branching of from 15% to 50%;
b. from 1.0 % to 25 % by weight of the concentrated surfactant blend of a buffering surfactant selected from the group consisting of: amphoteric surfactant, zwitterionic surfactant, and mixtures thereof; and

wherein the buffering surfactant is present at a level to provide the resultant concentrated surfactant blend with a reserve alkalinity of greater than 0.02, when measured as a 10% by weight solution of the concentrated surfactant blend in demineralized water at 20°C; and the resultant concentrated surfactant blend has a pH of from 7.1 to 10, measured as a 10% by weight solution of the concentrated surfactant blend in demineralized water at 20°C.

**15.** The concentrated surfactant blend according to claim 14, wherein the blend comprises:

a. from 40 % to 60 %, preferably from 45 % to 55 % by weight of the concentrated surfactant blend of the alkyl sulfated anionic surfactant; and
b. from 5.0 % to 20 %, preferably from 10 % to 15 % by weight of the concentrated surfactant blend of the buffering surfactant.

Europäisches Patentamt
European Patent Office
Office européen des brevets

# EUROPEAN SEARCH REPORT

Application Number
EP 22 20 2167

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/380901 A1 (BIILIAUW JAN JULIEN MARIE-LOUISE [BE] ET AL) 9 December 2021 (2021-12-09) * paragraph [70;161;163]; claims 1-20; tables 1,2a * ----- | 1-13 | INV. C11D1/75 C11D1/94 C11D11/04 C07C303/24 C07C305/06 |
| X | KR 2018 0039010 A (LION CORP [JP]) 17 April 2018 (2018-04-17) * paragraph [71;72;128;150;164;238]; claims 1,2; examples 18,19; compounds c-1,c-6 * ----- | 14,15 | |

TECHNICAL FIELDS SEARCHED (IPC)

C07C
C11D

The present search report has been drawn up for all claims

1

| Place of search | Date of completion of the search | Examiner |
|---|---|---|
| The Hague | 21 August 2023 | Douelle, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 20 2167

This annex lists the patent family members relating to the patent documents cited in the above-mentioned European search report. The members are as contained in the European Patent Office EDP file on
The European Patent Office is in no way liable for these particulars which are merely given for the purpose of information.

21-08-2023

| Patent document cited in search report | Publication date | Patent family member(s) | Publication date |
|---|---|---|---|
| US 2021380901 A1 | 09-12-2021 | EP 3919594 A1 | 08-12-2021 |
| | | JP 7264939 B2 | 25-04-2023 |
| | | JP 2021193174 A | 23-12-2021 |
| | | US 2021380901 A1 | 09-12-2021 |
| KR 20180039010 A | 17-04-2018 | CN 107922890 A | 17-04-2018 |
| | | KR 20180039010 A | 17-04-2018 |
| | | WO 2017026033 A1 | 16-02-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4477372 A
- US 4476044 A
- US 4476045 A
- WO 9418160 A
- EP 2964741 A
- WO 201472840 A
- WO 9738972 A
- WO 9404640 A
- WO 9105764 A
- US 20170158625 A
- GB 977281 A
- EP 3919594 A1
- WO 2007135645 A
- WO 2019108293 A1


### Non-patent literature cited in the description


- **W. HERMAN DE GROOT.** Sulf(on)ation Technology in the Detergent Industry. Springer-Science+Business Media, 1991